# EUROPEAN PATENT APPLICATION

(11) **EP 1 342 781 A1**
(43) Date of publication of application: **10.09.2003**
(21) Application number: 02005468.0
(22) Date of filing: 09.03.2002
(51) Int. Cl.: C12N 9/22, C12N 15/64, C12N 15/90

(54) **Recombinase fusion protein with enhanced cellular uptake**

(71) Applicant: ARTEMIS Pharmaceuticals GmbH, 51063 Köln (DE)
(72) Inventor: Edenhofer, Frank Oliver Stefan, 50374 Erfstadt (DE); Peitz, Michael, 50823 Köln (DE); Pfannkuche, Kurt, 50374 Erfstadt (DE); Rajewski, Klaus, 50931 Köln (DE)
(74) Representative: Helbing, Jörg, Dr. Dipl.-Chem.

(57) **Abstract**

The present invention relates to a fusion protein comprising a site-specific DNA recombinase domain such as Cre recombinase, and a domain containing a specific modified nuclear localization signal. The fusion protein may further comprise a protein transduction domain such as the hydrophobic FGF and basic TAT peptide. The specific nuclear localization signal - alone, or together with the protein transduction domain - promotes the cellular uptake of the recombinase. The fusion protein is a powerful tool for efficient genetic engineering of mammalian genomes. The invention further relates to DNA sequences coding for said fusion protein, methods for producing said fusion protein and its use as an agent for inducing gene targeting in a living organism or in cultured cells.

SEQUENCE LISTING

<110> ARTEMIS Pharmaceuticals GmbH

<120> Recombinase Fusion Protein with enhanced Cellular
      Uptake

<130> 020357ep/JH/ml

<140>
<141>

<160> 54

<170> PatentIn Ver. 2.1

<210> 1
<211> 1029
<212> DNA
<213> Artificial Sequence

<220>
<223> Description of Artificial Sequence: DNA sequence
      coding for Cre recombinase

<220>
<221> CDS
<222> (1)..(1026)

<400> 1
tcc aat tta ctg acc gta cac caa aat ttg cct gca tta ccg gtc gat     48
Ser Asn Leu Leu Thr Val His Gln Asn Leu Pro Ala Leu Pro Val Asp
  1               5                  10                  15

gca acg agt gat gag gtt cgc aag aac ctg atg gac atg ttc agg gat     96
Ala Thr Ser Asp Glu Val Arg Lys Asn Leu Met Asp Met Phe Arg Asp
             20                  25                  30

cgc cag gcg ttt tct gag cat acc tgg aaa atg ctt ctg tcc gtt tgc     144
Arg Gln Ala Phe Ser Glu His Thr Trp Lys Met Leu Leu Ser Val Cys
         35                  40                  45

cgg tcg tgg gcg gca tgg tgc aag ttg aat aac cgg aaa tgg ttt ccc     192
Arg Ser Trp Ala Ala Trp Cys Lys Leu Asn Asn Arg Lys Trp Phe Pro
     50                  55                  60

gca gaa cct gaa gat gtt cgc gat tat ctt cta tat ctt cag gcg cgc     240
Ala Glu Pro Glu Asp Val Arg Asp Tyr Leu Leu Tyr Leu Gln Ala Arg
 65                  70                  75                  80

ggt ctg gca gta aaa act atc cag caa cat ttg ggc cag cta aac atg     288
Gly Leu Ala Val Lys Thr Ile Gln Gln His Leu Gly Gln Leu Asn Met
                 85                  90                  95

ctt cat cgt cgg tcc ggg ctg cca cga cca agt gac agc aat gct gtt     336
Leu His Arg Arg Ser Gly Leu Pro Arg Pro Ser Asp Ser Asn Ala Val
            100                 105                 110

tca ctg gtt atg cgg cgg atc cga aaa gaa aac gtt gat gcc ggt gaa     384
Ser Leu Val Met Arg Arg Ile Arg Lys Glu Asn Val Asp Ala Gly Glu
        115                 120                 125

cgt gca aaa cag gct cta gcg ttc gaa cgc act gat ttc gac cag gtt 432
Arg Ala Lys Gln Ala Leu Ala Phe Glu Arg Thr Asp Phe Asp Gln Val
    130                 135                 140

cgt tca ctc atg gaa aat agc gat cgc tgc cag gat ata cgt aat ctg 480
Arg Ser Leu Met Glu Asn Ser Asp Arg Cys Gln Asp Ile Arg Asn Leu
145                 150                 155                 160

gca ttt ctg ggg att gct tat aac acc ctg tta cgt ata gcc gaa att 528
Ala Phe Leu Gly Ile Ala Tyr Asn Thr Leu Leu Arg Ile Ala Glu Ile
                165                 170                 175

gcc agg atc agg gtt aaa gat atc tca cgt act gac ggt ggg aga atg 576
Ala Arg Ile Arg Val Lys Asp Ile Ser Arg Thr Asp Gly Gly Arg Met
            180                 185                 190

tta atc cat att ggc aga acg aaa acg ctg gtt agc acc gca ggt gta 624
Leu Ile His Ile Gly Arg Thr Lys Thr Leu Val Ser Thr Ala Gly Val
        195                 200                 205

gag aag gca ctt agc ctg ggg gta act aaa ctg gtc gag cga tgg att 672
Glu Lys Ala Leu Ser Leu Gly Val Thr Lys Leu Val Glu Arg Trp Ile
    210                 215                 220

tcc gtc tct ggt gta gct gat gat ccg aat aac tac ctg ttt tgc cgg 720
Ser Val Ser Gly Val Ala Asp Asp Pro Asn Asn Tyr Leu Phe Cys Arg
225                 230                 235                 240

gtc aga aaa aat ggt gtt gcc gcg cca tct gcc acc agc cag cta tca 768
Val Arg Lys Asn Gly Val Ala Ala Pro Ser Ala Thr Ser Gln Leu Ser
                245                 250                 255

act cgc gcc ctg gaa ggg att ttt gaa gca act cat cga ttg att tac 816
Thr Arg Ala Leu Glu Gly Ile Phe Glu Ala Thr His Arg Leu Ile Tyr
            260                 265                 270

ggc gct aag gat gac tct ggt cag aga tac ctg gcc tgg tct gga cac 864
Gly Ala Lys Asp Asp Ser Gly Gln Arg Tyr Leu Ala Trp Ser Gly His
        275                 280                 285

agt gcc cgt gtc gga gcc gcg cga gat atg gcc cgc gct gga gtt tca 912
Ser Ala Arg Val Gly Ala Ala Arg Asp Met Ala Arg Ala Gly Val Ser
    290                 295                 300

ata ccg gag atc atg caa gct ggt ggc tgg acc aat gta aat att gtc 960
Ile Pro Glu Ile Met Gln Ala Gly Gly Trp Thr Asn Val Asn Ile Val
305                 310                 315                 320

atg aac tat atc cgt aac ctg gat agt gaa aca ggg gca atg gtg cgc 1008
Met Asn Tyr Ile Arg Asn Leu Asp Ser Glu Thr Gly Ala Met Val Arg
                325                 330                 335

ctg ctg gaa gat ggc gat tag 1029
Leu Leu Glu Asp Gly Asp
            340

<210> 2
<211> 342
<212> PRT
<213> Artificial Sequence
<223> Description of Artificial Sequence: DNA sequence

coding for Cre recombinase

<400> 2
Ser Asn Leu Leu Thr Val His Gln Asn Leu Pro Ala Leu Pro Val Asp
1               5                   10                  15

Ala Thr Ser Asp Glu Val Arg Lys Asn Leu Met Asp Met Phe Arg Asp
            20                  25                  30

Arg Gln Ala Phe Ser Glu His Thr Trp Lys Met Leu Leu Ser Val Cys
        35                  40                  45

Arg Ser Trp Ala Ala Trp Cys Lys Leu Asn Asn Arg Lys Trp Phe Pro
    50                  55                  60

Ala Glu Pro Glu Asp Val Arg Asp Tyr Leu Leu Tyr Leu Gln Ala Arg
65                  70                  75                  80

Gly Leu Ala Val Lys Thr Ile Gln Gln His Leu Gly Gln Leu Asn Met
                85                  90                  95

Leu His Arg Arg Ser Gly Leu Pro Arg Pro Ser Asp Ser Asn Ala Val
            100                 105                 110

Ser Leu Val Met Arg Arg Ile Arg Lys Glu Asn Val Asp Ala Gly Glu
        115                 120                 125

Arg Ala Lys Gln Ala Leu Ala Phe Glu Arg Thr Asp Phe Asp Gln Val
    130                 135                 140

Arg Ser Leu Met Glu Asn Ser Asp Arg Cys Gln Asp Ile Arg Asn Leu
145                 150                 155                 160

Ala Phe Leu Gly Ile Ala Tyr Asn Thr Leu Leu Arg Ile Ala Glu Ile
                165                 170                 175

Ala Arg Ile Arg Val Lys Asp Ile Ser Arg Thr Asp Gly Gly Arg Met
            180                 185                 190

Leu Ile His Ile Gly Arg Thr Lys Thr Leu Val Ser Thr Ala Gly Val
        195                 200                 205

Glu Lys Ala Leu Ser Leu Gly Val Thr Lys Leu Val Glu Arg Trp Ile
    210                 215                 220

Ser Val Ser Gly Val Ala Asp Asp Pro Asn Asn Tyr Leu Phe Cys Arg
225                 230                 235                 240

Val Arg Lys Asn Gly Val Ala Ala Pro Ser Ala Thr Ser Gln Leu Ser
                245                 250                 255

Thr Arg Ala Leu Glu Gly Ile Phe Glu Ala Thr His Arg Leu Ile Tyr
            260                 265                 270

Gly Ala Lys Asp Asp Ser Gly Gln Arg Tyr Leu Ala Trp Ser Gly His
        275                 280                 285

Ser Ala Arg Val Gly Ala Ala Arg Asp Met Ala Arg Ala Gly Val Ser
    290                 295                 300

Ile Pro Glu Ile Met Gln Ala Gly Gly Trp Thr Asn Val Asn Ile Val
305                 310                 315                 320

Met Asn Tyr Ile Arg Asn Leu Asp Ser Glu Thr Gly Ala Met Val Arg
            325                 330                 335

Leu Leu Glu Asp Gly Asp
        340

<210> 3
<211> 1272
<212> DNA
<213> Artificial Sequence

<220>
<223> Description of Artificial Sequence: DNA sequence
      coding for Flpe recombinase

<220>
<221> CDS
<222> (1)..(1266)

<400> 3
agt caa ttt gat ata tta tgt aaa aca cca cct aag gtc ctg gtt cgt      48
Ser Gln Phe Asp Ile Leu Cys Lys Thr Pro Pro Lys Val Leu Val Arg
  1               5                  10                  15

cag ttt gtg gaa agg ttt gaa aga cct tca ggg gaa aaa ata gca tca      96
Gln Phe Val Glu Arg Phe Glu Arg Pro Ser Gly Glu Lys Ile Ala Ser
             20                  25                  30

tgt gct gct gaa cta acc tat tta tgt tgg atg att act cat aac gga     144
Cys Ala Ala Glu Leu Thr Tyr Leu Cys Trp Met Ile Thr His Asn Gly
         35                  40                  45

aca gca atc aag aga gcc aca ttc atg agc tat aat act atc ata agc     192
Thr Ala Ile Lys Arg Ala Thr Phe Met Ser Tyr Asn Thr Ile Ile Ser
     50                  55                  60

aat tcg ctg agt ttc gat att gtc aac aaa tca ctc cag ttt aaa tac     240
Asn Ser Leu Ser Phe Asp Ile Val Asn Lys Ser Leu Gln Phe Lys Tyr
 65                  70                  75                  80

aag acg caa aaa gca aca att ctg gaa gcc tca tta aag aaa tta att     288
Lys Thr Gln Lys Ala Thr Ile Leu Glu Ala Ser Leu Lys Lys Leu Ile
                 85                  90                  95

cct gct tgg gaa ttt aca att att cct tac aat gga caa aaa cat caa     336
Pro Ala Trp Glu Phe Thr Ile Ile Pro Tyr Asn Gly Gln Lys His Gln
            100                 105                 110

tct gat atc act gat att gta agt agt ttg caa tta cag ttc gaa tca     384
Ser Asp Ile Thr Asp Ile Val Ser Ser Leu Gln Leu Gln Phe Glu Ser
        115                 120                 125

tcg gaa gaa gca gat aag gga aat agc cac agt aaa aaa atg ctt aaa     432
Ser Glu Glu Ala Asp Lys Gly Asn Ser His Ser Lys Lys Met Leu Lys
    130                 135                 140

gca ctt cta agt gag ggt gaa agc atc tgg gag atc act gag aaa ata     480
Ala Leu Leu Ser Glu Gly Glu Ser Ile Trp Glu Ile Thr Glu Lys Ile
145                 150                 155                 160

cta aat tcg ttt gag tat acc tcg aga ttt aca aaa aca aaa act tta 528
Leu Asn Ser Phe Glu Tyr Thr Ser Arg Phe Thr Lys Thr Lys Thr Leu
                165                 170                 175

tac caa ttc ctc ttc cta gct act ttc atc aat tgt gga aga ttc agc 576
Tyr Gln Phe Leu Phe Leu Ala Thr Phe Ile Asn Cys Gly Arg Phe Ser
            180                 185                 190

gat att aag aac gtt gat ccg aaa tca ttt aaa tta gtc caa aat aag 624
Asp Ile Lys Asn Val Asp Pro Lys Ser Phe Lys Leu Val Gln Asn Lys
        195                 200                 205

tat ctg gga gta ata atc cag tgt tta gtg aca gag aca aag aca agc 672
Tyr Leu Gly Val Ile Ile Gln Cys Leu Val Thr Glu Thr Lys Thr Ser
    210                 215                 220

gtt agt agg cac ata tac ttc ttt agc gca agg ggt agg atc gat cca 720
Val Ser Arg His Ile Tyr Phe Phe Ser Ala Arg Gly Arg Ile Asp Pro
225                 230                 235                 240

ctt gta tat ttg gat gaa ttt ttg agg aat tct gaa cca gtc cta aaa 768
Leu Val Tyr Leu Asp Glu Phe Leu Arg Asn Ser Glu Pro Val Leu Lys
                245                 250                 255

cga gta aat agg acc ggc aat tct tca agc aac aaa cag gaa tac caa 816
Arg Val Asn Arg Thr Gly Asn Ser Ser Ser Asn Lys Gln Glu Tyr Gln
            260                 265                 270

tta tta aaa gat aac tta gtc aga tcg tac aac aag gct ttg aag aaa 864
Leu Leu Lys Asp Asn Leu Val Arg Ser Tyr Asn Lys Ala Leu Lys Lys
        275                 280                 285

aat gcg cct tat cca atc ttt gct ata aag aat ggc cca aaa tct cac 912
Asn Ala Pro Tyr Pro Ile Phe Ala Ile Lys Asn Gly Pro Lys Ser His
    290                 295                 300

att gga aga cat ttg atg acc tca ttt ctg tca atg aag ggc cta acg 960
Ile Gly Arg His Leu Met Thr Ser Phe Leu Ser Met Lys Gly Leu Thr
305                 310                 315                 320

gag ttg act aat gtt gtg gga aat tgg agc gat aag cgt gct tct gcc 1008
Glu Leu Thr Asn Val Val Gly Asn Trp Ser Asp Lys Arg Ala Ser Ala
                325                 330                 335

gtg gcc agg aca acg tat act cat cag ata aca gca ata cct gat cac 1056
Val Ala Arg Thr Thr Tyr Thr His Gln Ile Thr Ala Ile Pro Asp His
            340                 345                 350

tac ttc gca cta gtt tct cgg tac tat gca tat gat cca ata tca aag 1104
Tyr Phe Ala Leu Val Ser Arg Tyr Tyr Ala Tyr Asp Pro Ile Ser Lys
        355                 360                 365

gaa atg ata gca ttg aag gat gag act aat cca att gag gag tgg cag 1152
Glu Met Ile Ala Leu Lys Asp Glu Thr Asn Pro Ile Glu Glu Trp Gln
    370                 375                 380

cat ata gaa cag cta aag ggt agt gct gaa gga agc ata cga tac ccc 1200
His Ile Glu Gln Leu Lys Gly Ser Ala Glu Gly Ser Ile Arg Tyr Pro
385                 390                 395                 400

gca tgg aat ggg ata ata tca cag gag gta cta gac tac ctt tca tcc 1248

Ala Trp Asn Gly Ile Ile Ser Gln Glu Val Leu Asp Tyr Leu Ser Ser
            405                 410                 415

tac ata aat aga cgc ata taatga                                    1272
Tyr Ile Asn Arg Arg Ile
        420

<210> 4
<211> 422
<212> PRT
<213> Artificial Sequence
<223> Description of Artificial Sequence: DNA sequence
      coding for Flpe recombinase

<400> 4
Ser Gln Phe Asp Ile Leu Cys Lys Thr Pro Pro Lys Val Leu Val Arg
  1               5                  10                  15

Gln Phe Val Glu Arg Phe Glu Arg Pro Ser Gly Glu Lys Ile Ala Ser
             20                  25                  30

Cys Ala Ala Glu Leu Thr Tyr Leu Cys Trp Met Ile Thr His Asn Gly
         35                  40                  45

Thr Ala Ile Lys Arg Ala Thr Phe Met Ser Tyr Asn Thr Ile Ile Ser
     50                  55                  60

Asn Ser Leu Ser Phe Asp Ile Val Asn Lys Ser Leu Gln Phe Lys Tyr
 65                  70                  75                  80

Lys Thr Gln Lys Ala Thr Ile Leu Glu Ala Ser Leu Lys Lys Leu Ile
                 85                  90                  95

Pro Ala Trp Glu Phe Thr Ile Ile Pro Tyr Asn Gly Gln Lys His Gln
            100                 105                 110

Ser Asp Ile Thr Asp Ile Val Ser Ser Leu Gln Leu Gln Phe Glu Ser
        115                 120                 125

Ser Glu Glu Ala Asp Lys Gly Asn Ser His Ser Lys Lys Met Leu Lys
    130                 135                 140

Ala Leu Leu Ser Glu Gly Glu Ser Ile Trp Glu Ile Thr Glu Lys Ile
145                 150                 155                 160

Leu Asn Ser Phe Glu Tyr Thr Ser Arg Phe Thr Lys Thr Lys Thr Leu
                165                 170                 175

Tyr Gln Phe Leu Phe Leu Ala Thr Phe Ile Asn Cys Gly Arg Phe Ser
            180                 185                 190

Asp Ile Lys Asn Val Asp Pro Lys Ser Phe Lys Leu Val Gln Asn Lys
        195                 200                 205

Tyr Leu Gly Val Ile Ile Gln Cys Leu Val Thr Glu Thr Lys Thr Ser
    210                 215                 220

Val Ser Arg His Ile Tyr Phe Phe Ser Ala Arg Gly Arg Ile Asp Pro
225                 230                 235                 240

Leu Val Tyr Leu Asp Glu Phe Leu Arg Asn Ser Glu Pro Val Leu Lys

245                 250                 255

Arg Val Asn Arg Thr Gly Asn Ser Ser Ser Asn Lys Gln Glu Tyr Gln
            260                 265                 270

Leu Leu Lys Asp Asn Leu Val Arg Ser Tyr Asn Lys Ala Leu Lys Lys
        275                 280                 285

Asn Ala Pro Tyr Pro Ile Phe Ala Ile Lys Asn Gly Pro Lys Ser His
    290                 295                 300

Ile Gly Arg His Leu Met Thr Ser Phe Leu Ser Met Lys Gly Leu Thr
305                 310                 315                 320

Glu Leu Thr Asn Val Val Gly Asn Trp Ser Asp Lys Arg Ala Ser Ala
                325                 330                 335

Val Ala Arg Thr Thr Tyr Thr His Gln Ile Thr Ala Ile Pro Asp His
            340                 345                 350

Tyr Phe Ala Leu Val Ser Arg Tyr Tyr Ala Tyr Asp Pro Ile Ser Lys
        355                 360                 365

Glu Met Ile Ala Leu Lys Asp Glu Thr Asn Pro Ile Glu Glu Trp Gln
    370                 375                 380

His Ile Glu Gln Leu Lys Gly Ser Ala Glu Gly Ser Ile Arg Tyr Pro
385                 390                 395                 400

Ala Trp Asn Gly Ile Ile Ser Gln Glu Val Leu Asp Tyr Leu Ser Ser
                405                 410                 415

Tyr Ile Asn Arg Arg Ile
            420

<210> 5
<211> 7
<212> PRT
<213> Simian virus 40

<400> 5
Pro Lys Lys Lys Arg Lys Val
 1               5

<210> 6
<211> 6
<212> PRT
<213> Artificial Sequence

<220>
<223> Description of Artificial Sequence: Modified NLS

<400> 6
Lys Lys Lys Arg Lys Val
 1               5

<210> 7
<211> 11

<212> PRT
<213> Human immunodeficiency virus

<400> 7
Tyr Gly Arg Lys Lys Arg Arg Gln Arg Arg Arg
 1               5                   10


<210> 8
<211> 10
<212> PRT
<213> Artificial Sequence

<220>
<223> Description of Artificial Sequence: synthetic TAT
      protein

<400> 8
Gly Arg Lys Lys Arg Arg Gln Arg Arg Arg
 1               5                   10


<210> 9
<211> 11
<212> PRT
<213> Artificial Sequence

<220>
<223> Description of Artificial Sequence:synthetic TAT
      protein

<400> 9
Ala Gly Arg Lys Lys Arg Arg Gln Arg Arg Arg
 1               5                   10


<210> 10
<211> 11
<212> PRT
<213> Artificial Sequence

<220>
<223> Description of Artificial Sequence: synthetic TAT
      protein

<400> 10
Tyr Ala Arg Lys Ala Arg Arg Gln Ala Arg Arg
 1               5                   10


<210> 11
<211> 11
<212> PRT
<213> Artificial Sequence

<220>
<223> Description of Artificial Sequence: synthetic TAT
      protein

<400> 11
Tyr Ala Arg Ala Ala Ala Arg Gln Ala Arg Ala
 1               5                   10

<210> 12
<211> 11
<212> PRT
<213> Artificial Sequence

<220>
<223> Description of Artificial Sequence: synthetic TAT
      protein

<400> 12
Tyr Ala Arg Ala Ala Arg Arg Ala Ala Arg Arg
 1               5                   10


<210> 13
<211> 11
<212> PRT
<213> Artificial Sequence

<220>
<223> Description of Artificial Sequence: synthetic TAT
      protein

<400> 13
Tyr Ala Arg Ala Ala Arg Arg Ala Ala Arg Ala
 1               5                   10


<210> 14
<211> 11
<212> PRT
<213> Artificial Sequence

<220>
<223> Description of Artificial Sequence: synthetic TAT
      protein

<400> 14
Tyr Ala Arg Arg Arg Arg Arg Arg Arg Arg Arg
 1               5                   10


<210> 15
<211> 11
<212> PRT
<213> Artificial Sequence

<220>
<223> Description of Artificial Sequence: synthetic TAT
      protein

<400> 15
Tyr Ala Ala Ala Arg Arg Arg Arg Arg Arg Arg
 1               5                   10


<210> 16
<211> 302
<212> PRT
<213> HSV

<400> 16
Met Thr Ser Arg Arg Ser Val Lys Ser Gly Pro Arg Glu Val Pro Arg
1               5                   10                  15

Asp Glu Tyr Glu Asp Leu Tyr Tyr Thr Pro Ser Ser Gly Met Ala Ser
            20                  25                  30

Pro Asp Ser Pro Pro Asp Thr Ser Arg Arg Gly Ala Leu Gln Thr Arg
        35                  40                  45

Ser Arg Gln Arg Gly Glu Val Arg Phe Val Gln Tyr Asp Glu Ser Asp
    50                  55                  60

Tyr Ala Leu Tyr Gly Gly Ser Ser Ser Glu Asp Asp Glu His Pro Glu
65                  70                  75                  80

Val Pro Arg Thr Arg Arg Pro Val Ser Gly Ala Val Leu Ser Gly Pro
                85                  90                  95

Gly Pro Ala Arg Ala Pro Pro Pro Pro Ala Gly Ser Gly Gly Ala Gly
            100                 105                 110

Arg Thr Pro Thr Thr Ala Pro Arg Ala Pro Arg Thr Gln Arg Val Ala
        115                 120                 125

Ser Lys Ala Pro Ala Ala Pro Ala Ala Glu Thr Thr Arg Gly Arg Lys
    130                 135                 140

Ser Ala Gln Pro Glu Ser Ala Ala Leu Pro Asp Ala Pro Ala Ser Thr
145                 150                 155                 160

Ala Pro Thr Arg Ser Lys Thr Pro Ala Gln Gly Leu Ala Arg Lys Leu
                165                 170                 175

His Phe Ser Thr Ala Pro Pro Asn Pro Asp Ala Pro Trp Thr Pro Arg
            180                 185                 190

Val Ala Gly Phe Asn Lys Arg Val Phe Cys Ala Ala Val Gly Arg Leu
        195                 200                 205

Ala Ala Met His Ala Arg Met Ala Ala Val Gln Leu Trp Asp Met Ser
    210                 215                 220

Arg Pro Arg Thr Asp Glu Asp Leu Asn Glu Leu Leu Gly Ile Thr Thr
225                 230                 235                 240

Ile Arg Val Thr Val Cys Glu Gly Lys Asn Leu Leu Gln Arg Ala Asn
                245                 250                 255

Glu Leu Val Asn Pro Asp Val Val Gln Asp Val Asp Ala Ala Thr Ala
            260                 265                 270

Thr Arg Gly Arg Ser Ala Ala Ser Arg Pro Thr Glu Arg Pro Arg Ala
        275                 280                 285

Pro Ala Arg Ser Ala Ser Arg Pro Arg Arg Pro Val Glu Gly
    290                 295                 300

<210> 17
<211> 142

<212> PRT
<213> HSV

<400> 17
Thr Ala Pro Thr Arg Ser Lys Thr Pro Ala Gln Gly Leu Ala Arg Lys
1               5                   10                  15

Leu His Phe Ser Thr Ala Pro Pro Asn Pro Asp Ala Pro Trp Thr Pro
            20                  25                  30

Arg Val Ala Gly Phe Asn Lys Arg Val Phe Cys Ala Ala Val Gly Arg
        35                  40                  45

Leu Ala Ala Met His Ala Arg Met Ala Ala Val Gln Leu Trp Asp Met
    50                  55                  60

Ser Arg Pro Arg Thr Asp Glu Asp Leu Asn Glu Leu Leu Gly Ile Thr
65                  70                  75                  80

Thr Ile Arg Val Thr Val Cys Glu Gly Lys Asn Leu Leu Gln Arg Ala
                85                  90                  95

Asn Glu Leu Val Asn Pro Asp Val Val Gln Asp Val Asp Ala Ala Thr
            100                 105                 110

Ala Thr Arg Gly Arg Ser Ala Ala Ser Arg Pro Thr Glu Arg Pro Arg
        115                 120                 125

Ala Pro Ala Arg Ser Ala Ser Arg Pro Arg Arg Pro Val Glu
    130                 135                 140


<210> 18
<211> 12
<212> PRT
<213> Karposi fibroblast growth factor

<400> 18
Ala Ala Val Leu Leu Pro Val Leu Leu Ala Ala Pro
1               5                   10


<210> 19
<211> 16
<212> PRT
<213> Antennapedia

<400> 19
Arg Gln Ile Lys Ile Trp Phe Gln Asn Arg Arg Met Lys Trp Lys Lys
1               5                   10                  15


<210> 20
<211> 34
<212> PRT
<213> HSV

<400> 20
Asp Ala Ala Thr Ala Thr Arg Gly Arg Ser Ala Ala Ser Arg Pro Thr
1               5                   10                  15

Glu Arg Pro Arg Ala Pro Ala Arg Ser Ala Ser Arg Pro Arg Arg Pro

            20                  25                  30

Val Glu

<210> 21
<211> 33
<212> DNA
<213> Human immunodeficiency virus

<400> 21
tacggccgca agaagcgccg ccaacgccgc cgc 33

<210> 22
<211> 90
<212> DNA
<213> Artificial Sequence

<220>
<223> Description of Artificial Sequence: Primer

<400> 22
cttggccatg ggcgctgcag gtcgcaagaa acgtcgccaa cgtcgccgtc cgcctgcagg 60
cactagtcag attttcgtca agactttgac 90

<210> 23
<211> 47
<212> DNA
<213> Artificial Sequence

<220>
<223> Description of Artificial Sequence: Primer

<400> 23
ccattactag ttgcgcccat accaccacgt agccttagca caagatg 47

<210> 24
<211> 34
<212> DNA
<213> Artificial Sequence

<220>
<223> Description of Artificial Sequence: Primer

<400> 24
cgtccaagct tgtccaattt actgaccgta cacc 34

<210> 25
<211> 34
<212> DNA
<213> Artificial Sequence

<220>
<223> Description of Artificial Sequence: Primer

<400> 25
ctgaactcga gaccatcgcc atcttccagc aggc 34

<210> 26
<211> 32
<212> DNA
<213> Artificial Sequence

<220>
<223> Description of Artificial Sequence: Primer

<400> 26
catggaagct tgaagaagaa gaggaaggtg tc                                32

<210> 27
<211> 48
<212> DNA
<213> Artificial Sequence

<220>
<223> Description of Artificial Sequence: Primer

<400> 27
gtagctttac ttccggcagt tctgttagcg ttgctggcac cgggtgca               48

<210> 28
<211> 48
<212> DNA
<213> Artificial Sequence

<220>
<223> Description of Artificial Sequence: Primer

<400> 28
cccggtgcca gcaacgctaa cagaactgcc ggaagtaaag ctactgca               48

<210> 29
<211> 32
<212> DNA
<213> Artificial Sequence

<220>
<223> Description of Artificial Sequence: Primer

<400> 29
ctaatctcga gctaatcgcc atcttccagc ag                                32

<210> 30
<211> 27
<212> DNA
<213> Artificial Sequence

<220>
<223> Description of Artificial Sequence: Primer

<400> 30
catgggccat caccatcacc atcacgg                                      27

<210> 31
<211> 27
<212> DNA
<213> Artificial Sequence

<220>
<223> Description of Artificial Sequence: Primer

<400> 31
catgccgtga tggtgatggt gatggcc                                          27


<210> 32
<211> 48
<212> DNA
<213> Artificial Sequence

<220>
<223> Description of Artificial Sequence: Primer

<400> 32
tcgagggtgc agctgtatta cttccggttc tgttagcggc accgtgat                   48


<210> 33
<211> 48
<212> DNA
<213> Artificial Sequence

<220>
<223> Description of Artificial Sequence: Primer

<400> 33
tcgagggtgc agctgtatta cttccggttc tgttagcggc accgtgat                   48


<210> 34
<211> 1094
<212> DNA
<213> Artificial Sequence

<220>
<223> Description of Artificial Sequence: vector CH

<220>
<221> CDS
<222> (3)..(1082)

<400> 34
cc atg ggc gct gca ggc act agt gta agc ttg tcc aat tta ctg acc    47
   Met Gly Ala Ala Gly Thr Ser Val Ser Leu Ser Asn Leu Leu Thr
   1               5                   10                  15

gta cac caa aat ttg cct gca tta ccg gtc gat gca acg agt gat gag    95
Val His Gln Asn Leu Pro Ala Leu Pro Val Asp Ala Thr Ser Asp Glu
                20                  25                  30

gtt cgc aag aac ctg atg gac atg ttc agg gat cgc cag gcg ttt tct    143
Val Arg Lys Asn Leu Met Asp Met Phe Arg Asp Arg Gln Ala Phe Ser
            35                  40                  45

gag cat acc tgg aaa atg ctt ctg tcc gtt tgc cgg tcg tgg gcg gca    191

Glu His Thr Trp Lys Met Leu Leu Ser Val Cys Arg Ser Trp Ala Ala
        50                  55                  60

tgg tgc aag ttg aat aac cgg aaa tgg ttt ccc gca gaa cct gaa gat 239
Trp Cys Lys Leu Asn Asn Arg Lys Trp Phe Pro Ala Glu Pro Glu Asp
    65                  70                  75

gtt cgc gat tat ctt cta tat ctt cag gcg cgc ggt ctg gca gta aaa 287
Val Arg Asp Tyr Leu Leu Tyr Leu Gln Ala Arg Gly Leu Ala Val Lys
80                  85                  90                  95

act atc cag caa cat ttg ggc cag cta aac atg ctt cat cgt cgg tcc 335
Thr Ile Gln Gln His Leu Gly Gln Leu Asn Met Leu His Arg Arg Ser
                100                 105                 110

ggg ctg cca cga cca agt gac agc aat gct gtt tca ctg gtt atg cgg 383
Gly Leu Pro Arg Pro Ser Asp Ser Asn Ala Val Ser Leu Val Met Arg
            115                 120                 125

cgg atc cga aaa gaa aac gtt gat gcc ggt gaa cgt gca aaa cag gct 431
Arg Ile Arg Lys Glu Asn Val Asp Ala Gly Glu Arg Ala Lys Gln Ala
        130                 135                 140

cta gcg ttc gaa cgc act gat ttc gac cag gtt cgt tca ctc atg gaa 479
Leu Ala Phe Glu Arg Thr Asp Phe Asp Gln Val Arg Ser Leu Met Glu
    145                 150                 155

aat agc gat cgc tgc cag gat ata cgt aat ctg gca ttt ctg ggg att 527
Asn Ser Asp Arg Cys Gln Asp Ile Arg Asn Leu Ala Phe Leu Gly Ile
160                 165                 170                 175

gct tat aac acc ctg tta cgt ata gcc gaa att gcc agg atc agg gtt 575
Ala Tyr Asn Thr Leu Leu Arg Ile Ala Glu Ile Ala Arg Ile Arg Val
                180                 185                 190

aaa gat atc tca cgt act gac ggt ggg aga atg tta atc cat att ggc 623
Lys Asp Ile Ser Arg Thr Asp Gly Gly Arg Met Leu Ile His Ile Gly
            195                 200                 205

aga acg aaa acg ctg gtt agc acc gca ggt gta gag aag gca ctt agc 671
Arg Thr Lys Thr Leu Val Ser Thr Ala Gly Val Glu Lys Ala Leu Ser
        210                 215                 220

ctg ggg gta act aaa ctg gtc gag cga tgg att tcc gtc tct ggt gta 719
Leu Gly Val Thr Lys Leu Val Glu Arg Trp Ile Ser Val Ser Gly Val
    225                 230                 235

gct gat gat ccg aat aac tac ctg ttt tgc cgg gtc aga aaa aat ggt 767
Ala Asp Asp Pro Asn Asn Tyr Leu Phe Cys Arg Val Arg Lys Asn Gly
240                 245                 250                 255

gtt gcc gcg cca tct gcc acc agc cag cta tca act cgc gcc ctg gaa 815
Val Ala Ala Pro Ser Ala Thr Ser Gln Leu Ser Thr Arg Ala Leu Glu
                260                 265                 270

ggg att ttt gaa gca act cat cga ttg att tac ggc gct aag gat gac 863
Gly Ile Phe Glu Ala Thr His Arg Leu Ile Tyr Gly Ala Lys Asp Asp
            275                 280                 285

tct ggt cag aga tac ctg gcc tgg tct gga cac agt gcc cgt gtc gga 911
Ser Gly Gln Arg Tyr Leu Ala Trp Ser Gly His Ser Ala Arg Val Gly
        290                 295                 300

gcc gcg cga gat atg gcc cgc gct gga gtt tca ata ccg gag atc atg    959
Ala Ala Arg Asp Met Ala Arg Ala Gly Val Ser Ile Pro Glu Ile Met
    305                 310                 315

caa gct ggt ggc tgg acc aat gta aat att gtc atg aac tat atc cgt    1007
Gln Ala Gly Gly Trp Thr Asn Val Asn Ile Val Met Asn Tyr Ile Arg
320                 325                 330                 335

aac ctg gat agt gaa aca ggg gca atg gtg cgc ctg ctg gaa gat ggc    1055
Asn Leu Asp Ser Glu Thr Gly Ala Met Val Arg Leu Leu Glu Asp Gly
                340                 345                 350

gat ggt ctc gag cac cac cat cac cat caccatcact aa                  1094
Asp Gly Leu Glu His His His His His
            355                 360

<210> 35
<211> 360
<212> PRT
<213> Artificial Sequence
<223> Description of Artificial Sequence: vector CH

<400> 35
Met Gly Ala Ala Gly Thr Ser Val Ser Leu Ser Asn Leu Leu Thr Val
  1               5                  10                  15

His Gln Asn Leu Pro Ala Leu Pro Val Asp Ala Thr Ser Asp Glu Val
             20                  25                  30

Arg Lys Asn Leu Met Asp Met Phe Arg Asp Arg Gln Ala Phe Ser Glu
         35                  40                  45

His Thr Trp Lys Met Leu Leu Ser Val Cys Arg Ser Trp Ala Ala Trp
     50                  55                  60

Cys Lys Leu Asn Asn Arg Lys Trp Phe Pro Ala Glu Pro Glu Asp Val
 65                  70                  75                  80

Arg Asp Tyr Leu Leu Tyr Leu Gln Ala Arg Gly Leu Ala Val Lys Thr
                 85                  90                  95

Ile Gln Gln His Leu Gly Gln Leu Asn Met Leu His Arg Arg Ser Gly
            100                 105                 110

Leu Pro Arg Pro Ser Asp Ser Asn Ala Val Ser Leu Val Met Arg Arg
        115                 120                 125

Ile Arg Lys Glu Asn Val Asp Ala Gly Glu Arg Ala Lys Gln Ala Leu
    130                 135                 140

Ala Phe Glu Arg Thr Asp Phe Asp Gln Val Arg Ser Leu Met Glu Asn
145                 150                 155                 160

Ser Asp Arg Cys Gln Asp Ile Arg Asn Leu Ala Phe Leu Gly Ile Ala
                165                 170                 175

Tyr Asn Thr Leu Leu Arg Ile Ala Glu Ile Ala Arg Ile Arg Val Lys
            180                 185                 190

Asp Ile Ser Arg Thr Asp Gly Gly Arg Met Leu Ile His Ile Gly Arg

195                 200                 205

Thr Lys Thr Leu Val Ser Thr Ala Gly Val Glu Lys Ala Leu Ser Leu
    210                 215                 220

Gly Val Thr Lys Leu Val Glu Arg Trp Ile Ser Val Ser Gly Val Ala
225                 230                 235                 240

Asp Asp Pro Asn Asn Tyr Leu Phe Cys Arg Val Arg Lys Asn Gly Val
                245                 250                 255

Ala Ala Pro Ser Ala Thr Ser Gln Leu Ser Thr Arg Ala Leu Glu Gly
            260                 265                 270

Ile Phe Glu Ala Thr His Arg Leu Ile Tyr Gly Ala Lys Asp Asp Ser
        275                 280                 285

Gly Gln Arg Tyr Leu Ala Trp Ser Gly His Ser Ala Arg Val Gly Ala
    290                 295                 300

Ala Arg Asp Met Ala Arg Ala Gly Val Ser Ile Pro Glu Ile Met Gln
305                 310                 315                 320

Ala Gly Gly Trp Thr Asn Val Asn Ile Val Met Asn Tyr Ile Arg Asn
                325                 330                 335

Leu Asp Ser Glu Thr Gly Ala Met Val Arg Leu Leu Glu Asp Gly Asp
            340                 345                 350

Gly Leu Glu His His His His His
        355                 360

<210> 36
<211> 1053
<212> DNA
<213> Artificial Sequence

<220>
<223> Description of Artificial Sequence: NLS-Cre

<220>
<221> CDS
<222> (1)..(1050)

<400> 36
atg ccc aag aag aag agg aag gtg tcc aat tta ctg acc gta cac caa      48
Met Pro Lys Lys Lys Arg Lys Val Ser Asn Leu Leu Thr Val His Gln
  1               5                  10                  15

aat ttg cct gca tta ccg gtc gat gca acg agt gat gag gtt cgc aag      96
Asn Leu Pro Ala Leu Pro Val Asp Ala Thr Ser Asp Glu Val Arg Lys
             20                  25                  30

aac ctg atg gac atg ttc agg gat cgc cag gcg ttt tct gag cat acc     144
Asn Leu Met Asp Met Phe Arg Asp Arg Gln Ala Phe Ser Glu His Thr
         35                  40                  45

tgg aaa atg ctt ctg tcc gtt tgc cgg tcg tgg gcg gca tgg tgc aag     192
Trp Lys Met Leu Leu Ser Val Cys Arg Ser Trp Ala Ala Trp Cys Lys
     50                  55                  60

ttg aat aac cgg aaa tgg ttt ccc gca gaa cct gaa gat gtt cgc gat 240
Leu Asn Asn Arg Lys Trp Phe Pro Ala Glu Pro Glu Asp Val Arg Asp
65                  70                  75                  80

tat ctt cta tat ctt cag gcg cgc ggt ctg gca gta aaa act atc cag 288
Tyr Leu Leu Tyr Leu Gln Ala Arg Gly Leu Ala Val Lys Thr Ile Gln
                85                  90                  95

caa cat ttg ggc cag cta aac atg ctt cat cgt cgg tcc ggg ctg cca 336
Gln His Leu Gly Gln Leu Asn Met Leu His Arg Arg Ser Gly Leu Pro
            100                 105                 110

cga cca agt gac agc aat gct gtt tca ctg gtt atg cgg cgg atc cga 384
Arg Pro Ser Asp Ser Asn Ala Val Ser Leu Val Met Arg Arg Ile Arg
        115                 120                 125

aaa gaa aac gtt gat gcc ggt gaa cgt gca aaa cag gct cta gcg ttc 432
Lys Glu Asn Val Asp Ala Gly Glu Arg Ala Lys Gln Ala Leu Ala Phe
    130                 135                 140

gaa cgc act gat ttc gac cag gtt cgt tca ctc atg gaa aat agc gat 480
Glu Arg Thr Asp Phe Asp Gln Val Arg Ser Leu Met Glu Asn Ser Asp
145                 150                 155                 160

cgc tgc cag gat ata cgt aat ctg gca ttt ctg ggg att gct tat aac 528
Arg Cys Gln Asp Ile Arg Asn Leu Ala Phe Leu Gly Ile Ala Tyr Asn
                165                 170                 175

acc ctg tta cgt ata gcc gaa att gcc agg atc agg gtt aaa gat atc 576
Thr Leu Leu Arg Ile Ala Glu Ile Ala Arg Ile Arg Val Lys Asp Ile
            180                 185                 190

tca cgt act gac ggt ggg aga atg tta atc cat att ggc aga acg aaa 624
Ser Arg Thr Asp Gly Gly Arg Met Leu Ile His Ile Gly Arg Thr Lys
        195                 200                 205

acg ctg gtt agc acc gca ggt gta gag aag gca ctt agc ctg ggg gta 672
Thr Leu Val Ser Thr Ala Gly Val Glu Lys Ala Leu Ser Leu Gly Val
    210                 215                 220

act aaa ctg gtc gag cga tgg att tcc gtc tct ggt gta gct gat gat 720
Thr Lys Leu Val Glu Arg Trp Ile Ser Val Ser Gly Val Ala Asp Asp
225                 230                 235                 240

ccg aat aac tac ctg ttt tgc cgg gtc aga aaa aat ggt gtt gcc gcg 768
Pro Asn Asn Tyr Leu Phe Cys Arg Val Arg Lys Asn Gly Val Ala Ala
                245                 250                 255

cca tct gcc acc agc cag cta tca act cgc gcc ctg gaa ggg att ttt 816
Pro Ser Ala Thr Ser Gln Leu Ser Thr Arg Ala Leu Glu Gly Ile Phe
            260                 265                 270

gaa gca act cat cga ttg att tac ggc gct aag gat gac tct ggt cag 864
Glu Ala Thr His Arg Leu Ile Tyr Gly Ala Lys Asp Asp Ser Gly Gln
        275                 280                 285

aga tac ctg gcc tgg tct gga cac agt gcc cgt gtc gga gcc gcg cga 912
Arg Tyr Leu Ala Trp Ser Gly His Ser Ala Arg Val Gly Ala Ala Arg
    290                 295                 300

gat atg gcc cgc gct gga gtt tca ata ccg gag atc atg caa gct ggt 960

Asp Met Ala Arg Ala Gly Val Ser Ile Pro Glu Ile Met Gln Ala Gly
305                 310                 315                 320

ggc tgg acc aat gta aat att gtc atg aac tat atc cgt aac ctg gat    1008
Gly Trp Thr Asn Val Asn Ile Val Met Asn Tyr Ile Arg Asn Leu Asp
                325                 330                 335

agt gaa aca ggg gca atg gtg cgc ctg ctg gaa gat ggc gat tag        1053
Ser Glu Thr Gly Ala Met Val Arg Leu Leu Glu Asp Gly Asp
            340                 345                 350

<210> 37
<211> 350
<212> PRT
<213> Artificial Sequence
<223> Description of Artificial Sequence: NLS-Cre

<400> 37
Met Pro Lys Lys Lys Arg Lys Val Ser Asn Leu Leu Thr Val His Gln
  1               5                  10                  15

Asn Leu Pro Ala Leu Pro Val Asp Ala Thr Ser Asp Glu Val Arg Lys
             20                  25                  30

Asn Leu Met Asp Met Phe Arg Asp Arg Gln Ala Phe Ser Glu His Thr
         35                  40                  45

Trp Lys Met Leu Leu Ser Val Cys Arg Ser Trp Ala Ala Trp Cys Lys
     50                  55                  60

Leu Asn Asn Arg Lys Trp Phe Pro Ala Glu Pro Glu Asp Val Arg Asp
 65                  70                  75                  80

Tyr Leu Leu Tyr Leu Gln Ala Arg Gly Leu Ala Val Lys Thr Ile Gln
                 85                  90                  95

Gln His Leu Gly Gln Leu Asn Met Leu His Arg Arg Ser Gly Leu Pro
            100                 105                 110

Arg Pro Ser Asp Ser Asn Ala Val Ser Leu Val Met Arg Arg Ile Arg
        115                 120                 125

Lys Glu Asn Val Asp Ala Gly Glu Arg Ala Lys Gln Ala Leu Ala Phe
    130                 135                 140

Glu Arg Thr Asp Phe Asp Gln Val Arg Ser Leu Met Glu Asn Ser Asp
145                 150                 155                 160

Arg Cys Gln Asp Ile Arg Asn Leu Ala Phe Leu Gly Ile Ala Tyr Asn
                165                 170                 175

Thr Leu Leu Arg Ile Ala Glu Ile Ala Arg Ile Arg Val Lys Asp Ile
            180                 185                 190

Ser Arg Thr Asp Gly Gly Arg Met Leu Ile His Ile Gly Arg Thr Lys
        195                 200                 205

Thr Leu Val Ser Thr Ala Gly Val Glu Lys Ala Leu Ser Leu Gly Val
    210                 215                 220

Thr Lys Leu Val Glu Arg Trp Ile Ser Val Ser Gly Val Ala Asp Asp

225                 230                 235                 240

Pro Asn Asn Tyr Leu Phe Cys Arg Val Arg Lys Asn Gly Val Ala Ala
                245                 250                 255

Pro Ser Ala Thr Ser Gln Leu Ser Thr Arg Ala Leu Glu Gly Ile Phe
            260                 265                 270

Glu Ala Thr His Arg Leu Ile Tyr Gly Ala Lys Asp Asp Ser Gly Gln
        275                 280                 285

Arg Tyr Leu Ala Trp Ser Gly His Ser Ala Arg Val Gly Ala Ala Arg
    290                 295                 300

Asp Met Ala Arg Ala Gly Val Ser Ile Pro Glu Ile Met Gln Ala Gly
305                 310                 315                 320

Gly Trp Thr Asn Val Asn Ile Val Met Asn Tyr Ile Arg Asn Leu Asp
                325                 330                 335

Ser Glu Thr Gly Ala Met Val Arg Leu Leu Glu Asp Gly Asp
            340                 345                 350

<210> 38
<211> 1112
<212> DNA
<213> Artificial Sequence

<220>
<223> Description of Artificial Sequence: HNC

<220>
<221> CDS
<222> (3)..(1103)

<400> 38
cc atg ggc cat cac cat cac cat cac ggc atg ggc gct gca ggc act      47
   Met Gly His His His His His His Gly Met Gly Ala Ala Gly Thr
     1               5                  10                  15

agt gta agc ttg aag aag aag agg aag gtg tcc aat tta ctg acc gta      95
Ser Val Ser Leu Lys Lys Lys Arg Lys Val Ser Asn Leu Leu Thr Val
                 20                  25                  30

cac caa aat ttg cct gca tta ccg gtc gat gca acg agt gat gag gtt     143
His Gln Asn Leu Pro Ala Leu Pro Val Asp Ala Thr Ser Asp Glu Val
             35                  40                  45

cgc aag aac ctg atg gac atg ttc agg gat cgc cag gcg ttt tct gag     191
Arg Lys Asn Leu Met Asp Met Phe Arg Asp Arg Gln Ala Phe Ser Glu
         50                  55                  60

cat acc tgg aaa atg ctt ctg tcc gtt tgc cgg tcg tgg gcg gca tgg     239
His Thr Trp Lys Met Leu Leu Ser Val Cys Arg Ser Trp Ala Ala Trp
     65                  70                  75

tgc aag ttg aat aac cgg aaa tgg ttt ccc gca gaa cct gaa gat gtt     287
Cys Lys Leu Asn Asn Arg Lys Trp Phe Pro Ala Glu Pro Glu Asp Val
 80                  85                  90                  95

cgc gat tat ctt cta tat ctt cag gcg cgc ggt ctg gca gta aaa act 335
Arg Asp Tyr Leu Leu Tyr Leu Gln Ala Arg Gly Leu Ala Val Lys Thr
                100                 105                 110

atc cag caa cat ttg ggc cag cta aac atg ctt cat cgt cgg tcc ggg 383
Ile Gln Gln His Leu Gly Gln Leu Asn Met Leu His Arg Arg Ser Gly
            115                 120                 125

ctg cca cga cca agt gac agc aat gct gtt tca ctg gtt atg cgg cgg 431
Leu Pro Arg Pro Ser Asp Ser Asn Ala Val Ser Leu Val Met Arg Arg
        130                 135                 140

atc cga aaa gaa aac gtt gat gcc ggt gaa cgt gca aaa cag gct cta 479
Ile Arg Lys Glu Asn Val Asp Ala Gly Glu Arg Ala Lys Gln Ala Leu
    145                 150                 155

gcg ttc gaa cgc act gat ttc gac cag gtt cgt tca ctc atg gaa aat 527
Ala Phe Glu Arg Thr Asp Phe Asp Gln Val Arg Ser Leu Met Glu Asn
160                 165                 170                 175

agc gat cgc tgc cag gat ata cgt aat ctg gca ttt ctg ggg att gct 575
Ser Asp Arg Cys Gln Asp Ile Arg Asn Leu Ala Phe Leu Gly Ile Ala
                180                 185                 190

tat aac acc ctg tta cgt ata gcc gaa att gcc agg atc agg gtt aaa 623
Tyr Asn Thr Leu Leu Arg Ile Ala Glu Ile Ala Arg Ile Arg Val Lys
            195                 200                 205

gat atc tca cgt act gac ggt ggg aga atg tta atc cat att ggc aga 671
Asp Ile Ser Arg Thr Asp Gly Gly Arg Met Leu Ile His Ile Gly Arg
        210                 215                 220

acg aaa acg ctg gtt agc acc gca ggt gta gag aag gca ctt agc ctg 719
Thr Lys Thr Leu Val Ser Thr Ala Gly Val Glu Lys Ala Leu Ser Leu
    225                 230                 235

ggg gta act aaa ctg gtc gag cga tgg att tcc gtc tct ggt gta gct 767
Gly Val Thr Lys Leu Val Glu Arg Trp Ile Ser Val Ser Gly Val Ala
240                 245                 250                 255

gat gat ccg aat aac tac ctg ttt tgc cgg gtc aga aaa aat ggt gtt 815
Asp Asp Pro Asn Asn Tyr Leu Phe Cys Arg Val Arg Lys Asn Gly Val
                260                 265                 270

gcc gcg cca tct gcc acc agc cag cta tca act cgc gcc ctg gaa ggg 863
Ala Ala Pro Ser Ala Thr Ser Gln Leu Ser Thr Arg Ala Leu Glu Gly
            275                 280                 285

att ttt gaa gca act cat cga ttg att tac ggc gct aag gat gac tct 911
Ile Phe Glu Ala Thr His Arg Leu Ile Tyr Gly Ala Lys Asp Asp Ser
        290                 295                 300

ggt cag aga tac ctg gcc tgg tct gga cac agt gcc cgt gtc gga gcc 959
Gly Gln Arg Tyr Leu Ala Trp Ser Gly His Ser Ala Arg Val Gly Ala
    305                 310                 315

gcg cga gat atg gcc cgc gct gga gtt tca ata ccg gag atc atg caa 1007
Ala Arg Asp Met Ala Arg Ala Gly Val Ser Ile Pro Glu Ile Met Gln
320                 325                 330                 335

gct ggt ggc tgg acc aat gta aat att gtc atg aac tat atc cgt aac 1055
Ala Gly Gly Trp Thr Asn Val Asn Ile Val Met Asn Tyr Ile Arg Asn

340                 345                 350

ctg gat agt gaa aca ggg gca atg gtg cgc ctg ctg gaa gat ggc gat    1103
Leu Asp Ser Glu Thr Gly Ala Met Val Arg Leu Leu Glu Asp Gly Asp
        355                 360                 365

tagctcgag                                                          1112

<210> 39
<211> 367
<212> PRT
<213> Artificial Sequence
<223> Description of Artificial Sequence: HNC

<400> 39
Met Gly His His His His His His Gly Met Gly Ala Ala Gly Thr Ser
 1               5                  10                  15

Val Ser Leu Lys Lys Lys Arg Lys Val Ser Asn Leu Leu Thr Val His
            20                  25                  30

Gln Asn Leu Pro Ala Leu Pro Val Asp Ala Thr Ser Asp Glu Val Arg
        35                  40                  45

Lys Asn Leu Met Asp Met Phe Arg Asp Arg Gln Ala Phe Ser Glu His
    50                  55                  60

Thr Trp Lys Met Leu Leu Ser Val Cys Arg Ser Trp Ala Ala Trp Cys
65                  70                  75                  80

Lys Leu Asn Asn Arg Lys Trp Phe Pro Ala Glu Pro Glu Asp Val Arg
                85                  90                  95

Asp Tyr Leu Leu Tyr Leu Gln Ala Arg Gly Leu Ala Val Lys Thr Ile
            100                 105                 110

Gln Gln His Leu Gly Gln Leu Asn Met Leu His Arg Arg Ser Gly Leu
        115                 120                 125

Pro Arg Pro Ser Asp Ser Asn Ala Val Ser Leu Val Met Arg Arg Ile
    130                 135                 140

Arg Lys Glu Asn Val Asp Ala Gly Glu Arg Ala Lys Gln Ala Leu Ala
145                 150                 155                 160

Phe Glu Arg Thr Asp Phe Asp Gln Val Arg Ser Leu Met Glu Asn Ser
                165                 170                 175

Asp Arg Cys Gln Asp Ile Arg Asn Leu Ala Phe Leu Gly Ile Ala Tyr
            180                 185                 190

Asn Thr Leu Leu Arg Ile Ala Glu Ile Ala Arg Ile Arg Val Lys Asp
        195                 200                 205

Ile Ser Arg Thr Asp Gly Gly Arg Met Leu Ile His Ile Gly Arg Thr
    210                 215                 220

Lys Thr Leu Val Ser Thr Ala Gly Val Glu Lys Ala Leu Ser Leu Gly
225                 230                 235                 240

Val Thr Lys Leu Val Glu Arg Trp Ile Ser Val Ser Gly Val Ala Asp

245                 250                 255

Asp Pro Asn Asn Tyr Leu Phe Cys Arg Val Arg Lys Asn Gly Val Ala
            260                 265                 270

Ala Pro Ser Ala Thr Ser Gln Leu Ser Thr Arg Ala Leu Glu Gly Ile
        275                 280                 285

Phe Glu Ala Thr His Arg Leu Ile Tyr Gly Ala Lys Asp Asp Ser Gly
    290                 295                 300

Gln Arg Tyr Leu Ala Trp Ser Gly His Ser Ala Arg Val Gly Ala Ala
305                 310                 315                 320

Arg Asp Met Ala Arg Ala Gly Val Ser Ile Pro Glu Ile Met Gln Ala
                325                 330                 335

Gly Gly Trp Thr Asn Val Asn Ile Val Met Asn Tyr Ile Arg Asn Leu
            340                 345                 350

Asp Ser Glu Thr Gly Ala Met Val Arg Leu Leu Glu Asp Gly Asp
        355                 360                 365

<210> 40
<211> 1151
<212> DNA
<213> Artificial Sequence

<220>
<223> Description of Artificial Sequence: HTNC

<220>
<221> CDS
<222> (3)..(1142)

<400> 40
cc atg ggc cat cac cat cac cat cac ggc atg ggc gct gca ggt cgc      47
   Met Gly His His His His His His Gly Met Gly Ala Ala Gly Arg
     1               5                  10                  15

aag aaa cgt cgc caa cgt cgc cgt ccg cct gca ggc act agt gta agc      95
Lys Lys Arg Arg Gln Arg Arg Arg Pro Pro Ala Gly Thr Ser Val Ser
                 20                  25                  30

ttg aag aag aag agg aag gtg tcc aat tta ctg acc gta cac caa aat     143
Leu Lys Lys Lys Arg Lys Val Ser Asn Leu Leu Thr Val His Gln Asn
             35                  40                  45

ttg cct gca tta ccg gtc gat gca acg agt gat gag gtt cgc aag aac     191
Leu Pro Ala Leu Pro Val Asp Ala Thr Ser Asp Glu Val Arg Lys Asn
         50                  55                  60

ctg atg gac atg ttc agg gat cgc cag gcg ttt tct gag cat acc tgg     239
Leu Met Asp Met Phe Arg Asp Arg Gln Ala Phe Ser Glu His Thr Trp
     65                  70                  75

aaa atg ctt ctg tcc gtt tgc cgg tcg tgg gcg gca tgg tgc aag ttg     287
Lys Met Leu Leu Ser Val Cys Arg Ser Trp Ala Ala Trp Cys Lys Leu
 80                  85                  90                  95

aat aac cgg aaa tgg ttt ccc gca gaa cct gaa gat gtt cgc gat tat 335
Asn Asn Arg Lys Trp Phe Pro Ala Glu Pro Glu Asp Val Arg Asp Tyr
                100                 105                 110

ctt cta tat ctt cag gcg cgc ggt ctg gca gta aaa act atc cag caa 383
Leu Leu Tyr Leu Gln Ala Arg Gly Leu Ala Val Lys Thr Ile Gln Gln
            115                 120                 125

cat ttg ggc cag cta aac atg ctt cat cgt cgg tcc ggg ctg cca cga 431
His Leu Gly Gln Leu Asn Met Leu His Arg Arg Ser Gly Leu Pro Arg
        130                 135                 140

cca agt gac agc aat gct gtt tca ctg gtt atg cgg cgg atc cga aaa 479
Pro Ser Asp Ser Asn Ala Val Ser Leu Val Met Arg Arg Ile Arg Lys
    145                 150                 155

gaa aac gtt gat gcc ggt gaa cgt gca aaa cag gct cta gcg ttc gaa 527
Glu Asn Val Asp Ala Gly Glu Arg Ala Lys Gln Ala Leu Ala Phe Glu
160                 165                 170                 175

cgc act gat ttc gac cag gtt cgt tca ctc atg gaa aat agc gat cgc 575
Arg Thr Asp Phe Asp Gln Val Arg Ser Leu Met Glu Asn Ser Asp Arg
                180                 185                 190

tgc cag gat ata cgt aat ctg gca ttt ctg ggg att gct tat aac acc 623
Cys Gln Asp Ile Arg Asn Leu Ala Phe Leu Gly Ile Ala Tyr Asn Thr
            195                 200                 205

ctg tta cgt ata gcc gaa att gcc agg atc agg gtt aaa gat atc tca 671
Leu Leu Arg Ile Ala Glu Ile Ala Arg Ile Arg Val Lys Asp Ile Ser
        210                 215                 220

cgt act gac ggt ggg aga atg tta atc cat att ggc aga acg aaa acg 719
Arg Thr Asp Gly Gly Arg Met Leu Ile His Ile Gly Arg Thr Lys Thr
    225                 230                 235

ctg gtt agc acc gca ggt gta gag aag gca ctt agc ctg ggg gta act 767
Leu Val Ser Thr Ala Gly Val Glu Lys Ala Leu Ser Leu Gly Val Thr
240                 245                 250                 255

aaa ctg gtc gag cga tgg att tcc gtc tct ggt gta gct gat gat ccg 815
Lys Leu Val Glu Arg Trp Ile Ser Val Ser Gly Val Ala Asp Asp Pro
                260                 265                 270

aat aac tac ctg ttt tgc cgg gtc aga aaa aat ggt gtt gcc gcg cca 863
Asn Asn Tyr Leu Phe Cys Arg Val Arg Lys Asn Gly Val Ala Ala Pro
            275                 280                 285

tct gcc acc agc cag cta tca act cgc gcc ctg gaa ggg att ttt gaa 911
Ser Ala Thr Ser Gln Leu Ser Thr Arg Ala Leu Glu Gly Ile Phe Glu
        290                 295                 300

gca act cat cga ttg att tac ggc gct aag gat gac tct ggt cag aga 959
Ala Thr His Arg Leu Ile Tyr Gly Ala Lys Asp Asp Ser Gly Gln Arg
    305                 310                 315

tac ctg gcc tgg tct gga cac agt gcc cgt gtc gga gcc gcg cga gat 1007
Tyr Leu Ala Trp Ser Gly His Ser Ala Arg Val Gly Ala Ala Arg Asp
320                 325                 330                 335

atg gcc cgc gct gga gtt tca ata ccg gag atc atg caa gct ggt ggc 1055
Met Ala Arg Ala Gly Val Ser Ile Pro Glu Ile Met Gln Ala Gly Gly

340                345                350

tgg acc aat gta aat att gtc atg aac tat atc cgt aac ctg gat agt    1103
Trp Thr Asn Val Asn Ile Val Met Asn Tyr Ile Arg Asn Leu Asp Ser
        355                 360                 365

gaa aca ggg gca atg gtg cgc ctg ctg gaa gat ggc gat tagctcgag    1151
Glu Thr Gly Ala Met Val Arg Leu Leu Glu Asp Gly Asp
    370                 375                 380

<210> 41
<211> 380
<212> PRT
<213> Artificial Sequence
<223> Description of Artificial Sequence: HTNC

<400> 41
Met Gly His His His His His His Gly Met Gly Ala Ala Gly Arg Lys
 1               5                  10                  15

Lys Arg Arg Gln Arg Arg Arg Pro Pro Ala Gly Thr Ser Val Ser Leu
            20                  25                  30

Lys Lys Lys Arg Lys Val Ser Asn Leu Leu Thr Val His Gln Asn Leu
        35                  40                  45

Pro Ala Leu Pro Val Asp Ala Thr Ser Asp Glu Val Arg Lys Asn Leu
    50                  55                  60

Met Asp Met Phe Arg Asp Arg Gln Ala Phe Ser Glu His Thr Trp Lys
65                  70                  75                  80

Met Leu Leu Ser Val Cys Arg Ser Trp Ala Ala Trp Cys Lys Leu Asn
                85                  90                  95

Asn Arg Lys Trp Phe Pro Ala Glu Pro Glu Asp Val Arg Asp Tyr Leu
            100                 105                 110

Leu Tyr Leu Gln Ala Arg Gly Leu Ala Val Lys Thr Ile Gln Gln His
        115                 120                 125

Leu Gly Gln Leu Asn Met Leu His Arg Arg Ser Gly Leu Pro Arg Pro
    130                 135                 140

Ser Asp Ser Asn Ala Val Ser Leu Val Met Arg Arg Ile Arg Lys Glu
145                 150                 155                 160

Asn Val Asp Ala Gly Glu Arg Ala Lys Gln Ala Leu Ala Phe Glu Arg
                165                 170                 175

Thr Asp Phe Asp Gln Val Arg Ser Leu Met Glu Asn Ser Asp Arg Cys
            180                 185                 190

Gln Asp Ile Arg Asn Leu Ala Phe Leu Gly Ile Ala Tyr Asn Thr Leu
        195                 200                 205

Leu Arg Ile Ala Glu Ile Ala Arg Ile Arg Val Lys Asp Ile Ser Arg
    210                 215                 220

Thr Asp Gly Gly Arg Met Leu Ile His Ile Gly Arg Thr Lys Thr Leu
225                 230                 235                 240

Val Ser Thr Ala Gly Val Glu Lys Ala Leu Ser Leu Gly Val Thr Lys
            245                 250                 255

Leu Val Glu Arg Trp Ile Ser Val Ser Gly Val Ala Asp Asp Pro Asn
        260                 265                 270

Asn Tyr Leu Phe Cys Arg Val Arg Lys Asn Gly Val Ala Ala Pro Ser
    275                 280                 285

Ala Thr Ser Gln Leu Ser Thr Arg Ala Leu Glu Gly Ile Phe Glu Ala
    290                 295                 300

Thr His Arg Leu Ile Tyr Gly Ala Lys Asp Asp Ser Gly Gln Arg Tyr
305                 310                 315                 320

Leu Ala Trp Ser Gly His Ser Ala Arg Val Gly Ala Ala Arg Asp Met
                325                 330                 335

Ala Arg Ala Gly Val Ser Ile Pro Glu Ile Met Gln Ala Gly Gly Trp
            340                 345                 350

Thr Asn Val Asn Ile Val Met Asn Tyr Ile Arg Asn Leu Asp Ser Glu
        355                 360                 365

Thr Gly Ala Met Val Arg Leu Leu Glu Asp Gly Asp
    370                 375                 380

<210> 42
<211> 16
<212> PRT
<213> Simian virus 40

<400> 42
Lys Arg Pro Ala Ala Thr Lys Lys Ala Gly Gln Ala Lys Lys Lys Lys
1               5                   10                  15

<210> 43
<211> 42
<212> DNA
<213> Human immunodeficiency virus

<220>
<221> CDS
<222> (4)..(42)

<400> 43
atg ggc tac ggc cgc aag aag cgc cgc caa cgc cgc cgc ggc          42
    Gly Tyr Gly Arg Lys Lys Arg Arg Gln Arg Arg Arg Gly
    1               5                   10

<210> 44
<211> 13
<212> PRT
<213> Human immunodeficiency virus

<400> 44
Gly Tyr Gly Arg Lys Lys Arg Arg Gln Arg Arg Arg Gly

1                    5                        10

<210> 45
<211> 6
<212> PRT
<213> Artificial Sequence

<220>
<223> Description of Artificial Sequence: modified NLS

<400> 45
Lys Lys Lys Lys Lys Val
 1               5

<210> 46
<211> 6
<212> PRT
<213> Artificial Sequence

<220>
<223> Description of Artificial Sequence: modified NLS

<400> 46
Lys Lys Lys Lys Lys Lys
 1               5

<210> 47
<211> 6
<212> PRT
<213> Artificial Sequence

<220>
<223> Description of Artificial Sequence: modified NLS

<400> 47
Lys Lys Arg Arg Lys Val
 1               5

<210> 48
<211> 6
<212> PRT
<213> Artificial Sequence

<220>
<223> Description of Artificial Sequence: modified NLS

<400> 48
Lys Arg Arg Arg Lys Val
 1               5

<210> 49
<211> 6
<212> PRT
<213> Artificial Sequence

<220>

<212> PRT
<213> Artificial Sequence

<220>
<223> Description of Artificial Sequence: modified NLS

<400> 49
Arg Arg Arg Arg Lys Val
1               5


<210> 50
<211> 6
<212> PRT
<213> Artificial Sequence

<220>
<223> Description of Artificial Sequence: modified NLS

<400> 50
Arg Arg Arg Arg Arg Val
1               5


<210> 51
<211> 6
<212> PRT
<213> Artificial Sequence

<220>
<223> Description of Artificial Sequence: modified NLS

<400> 51
Arg Arg Arg Arg Arg Arg
1               5


<210> 52
<211> 8
<212> PRT
<213> Simian virus 40

<400> 52
Ala Pro Thr Lys Arg Lys Gly Ser
1               5


<210> 53
<211> 7
<212> PRT
<213> Simian virus 40

<400> 53
Pro Asn Lys Lys Lys Arg Lys
1               5


<210> 54
<211> 6
<212> PRT
<213> Artificial Sequence

<220>
<223> Description of Artificial Sequence: modified NLS

<400> 54
Asn Lys Lys Lys Arg Lys
 1               5

## Description

The present invention relates to a fusion protein comprising a site-specific DNA recombinase domain such as Cre recombinase, and a domain containing a specific modified nuclear localization signal. The fusion protein may further comprise a protein transduction domain such as the hydrophobic FGF and basic TAT peptide. The specific nuclear localization signal - alone, or together with the protein transduction domain - promotes the cellular uptake of the recombinase. The fusion protein is a powerful tool for efficient genetic engineering of mammalian genomes. The invention further relates to DNA sequences coding for said fusion protein, methods for producing said fusion protein and its use as an agent for inducing gene targeting in living organisms or in cell cultures.

### Summary of the Background Art

Conditional mutagenesis is a powerful tool to analyze gene functions in mammalian cells. The site-specific recombinase Cre can be used to recombine loxP-modified alleles under temporal and spatial control. However, the efficient delivery of biologically active Cre recombinase to living cells represents a limiting factor. In this study we compared the potential of a hydrophobic 12 amino acid peptide modified from Kaposi fibroblast growth factor (FGF) with a basic peptide derived from HIV-TAT to promote cellular uptake of recombinant Cre. E.g. WO01/4983 discloses a fusion protein comprising a site-specific DNA recombinase domain (such as Cre or Flpe) and a protein transduction domain (such as TAT or VP22). Said fusion protein may further contain a nuclear localization signal. The fusion protein is suitable for inducing target gene alterations in a living organism or cell culture, wherein said living organism carries at least one or more recognition sites for said site-specific DNA recombinase integrated in its genome.

Conditional mutagenesis in mammalian cells has become an important means for the analysis of gene function in vivo (Rajewsky, K. et al. J. Clin. Invest. 98, 51-53 (1996); Nagy, A. Genesis 26, 99-109 (2000); Lewandoski, M. Nat. Rev. Genet. 2, 743-755 (2001)). Site-specific recombinases such as the bacteriophage P1 recombinase Cre have been used to gain control over the mutation in a spatial (Orban, P.C. et al., Proc. Natl. Acad. Sci. USA 89, 6861-6865 (1992); Lakso, M. et al., Proc. Natl. Acad. Sci. USA, 89, 6232-6236 (1992); Gu, H. et al., Science 265, 103-106 (1994)) or/and temporal manner (Kühn, R. et al., Science 269, 1427-1429 (1995); Kellendonk, C. et al. Nucleic Acids Res. 24, 1404-1411 (1996)). An increasing number of studies have demonstrated the efficacy of Cre-mediated conditional mutagenesis in mice and cell lines (Kellendonk, C. et al., J. Mol.. Biol. 285, 175-182 (1999); Kulkarni, R.N. et al. Cell 96, 329-339 (1999); Li, M. et al., Nature 407, 633-636 (2000); Maruyama, M. et al., Nature 407, 636-642 (2000)). Usually a mouse or cell line is generated, in which an essential part of the gene of interest is flanked by two loxP sites. The loxP sites represent Cre recombination recognition sites and can be used to delete the respective gene segment upon Cre recombination, resulting in a conditioned inactivation or mutation of the gene of interest. In this context the delivery of biologically active Cre recombinase to mammalian cells either *in vivo* or *in vitro* is a limiting step. Four approaches have been used to deliver Cre into living cells: transfection, viral infection, transgenics and protein transduction.

### (i) Delivery of Cre by transfection:

A Cre encoding sequence is placed downstream of an appropriate promoter of an expression plasmid enabling transcription in the host cell. The plasmid can be delivered into the cells either by physical methods such as electroporation (Torres, R.M., Kühn, R., Laboratory protocols for conditional gene targeting (Oxford University Press, Oxford) (1997)) or by chemical methods such as Ca-phosphate precipitation (Kellendonk, C. et al. Nucleic Acids Res. 24, 1404-1411 (1996)) or lipofection (Wunderlich, F.T. et al., Nucleic Acids Res. 29, e47 (2001)) using non-ionic or cationic synthetic lipids. After cellular uptake of the Cre expression plasmid the gene is transcribed and the corresponding mRNA translated into Cre protein.

### (ii) Delivery of Cre by viral infection:

A promoter/Cre combination is cloned into a viral vector and the gene construct enters the cell by viral infection (Rohlmann, A. et al. Nat. Biotechnol. 14, 1562-1565 (1996); Shibata, H. et al., Science 278, 120-123 (1997)). As above transcription and translation takes place in the host cell. Viral Cre-delivery has been used in vitro as well as in vivo

### (iii) Expression of Cre from transgenes:

In contrast to the above mentioned methods the genetic information for the expression of the Cre recombinase is already in the cell by using Cre-transgenic cells or animals. The Cre transgene (promoter and Cre encoding sequence) can be introduced either randomly into the genome by injection into the male pronucleus of a mouse zygote ('classical transgenics') or at a distinct site by homologous recombination in embryonic stem cells ('knock-in').
The cell-specificity of the promoter confers spatial control over the mutation since Cre is only active in a distinct cell-type. In order to gain also temporal control over the mutation Cre recombinase activity can be regulated. Thereby Cre activity is induced by application of an exogenous inducer. Induction can be carried out either at the transcriptional level (e.g. Mx-Cre (Kühn, R. et al., Science 269, 1427-1429 (1995)) or tetracycline-controlled Cre expression (Utomo, A.R. et al., Nat. Biotechnol. 17, 1091-1096 (1999)) or post-translational level employing fusion proteins of Cre with mutated ligand-binding domains of steroid receptors (Kellendonk, C. et al. Nucleic Acids Res. 24, 1404-1411 (1996)).

Although a number of studies demonstrated the efficacy of Cre-mediated inducible mutagenesis, still the system could profit substantially from technical advance concerning three major aspects: leakiness of the system before induction, efficiency of induced recombination, and requirement of extensive mouse breeding causing the experiments to be time consuming and costly. The leakiness of the system represents a critical factor because a Cre recombinase which is undesirably active before induction often leads to unwanted side effects such as mosaic recombination and/or selection of recombined or non-recombined cells both *in vivo* and *in vitro* (Kellendonk, C. et al., J. Mol.. Biol. 285, 175-182 (1999); Minamino, T. et al., Circ. Res. 88, 587-592 (2001); Fuhrmann-Benzakein, E. et al., Nucleic Acids Res. 28, e99 (2000)). Moreover, the widely used inducers interferon, hydroxy-tamoxifen and doxycycline are known to display toxic side-effects (Vasioukhin, V. et al., Proc. Natl. Acad. Sci. USA 96, 8551-8556 (1999); Danielian, P.S. et al., Curr. Biol. 8, 1323-1326 (1998)) and/or induce also unwanted physiological effects which may interfere with the experimental phenotype of the conditional mutation to be analyzed (Kuzin, I.I. et al., Int. Immunol. 13, 921-931 (2001); Lin, Q. et al., J. Exp. Med. 187, 79-87 (1998)).
In cultured cells Cre-mediated recombination is limited by transfection efficiencies and putative toxicity of the protein (Torres, R.M., Kühn, R., Laboratory protocols for conditional gene targeting (Oxford University Press, Oxford) (1997); Loonstra, A. et al., Proc. Natl. Acad. Sci. USA 98, 209-214 (9001); Silver, D.P., Livingston, D.M., Mol. Cell. 8, 233-43 (2001)). Thus traditional delivery of Cre - either by Cre transgenics, viral vectors or transfection - represents a limiting step of conditional mutagenesis employing Cre/loxP technology.

### (iv) Delivery of Cre by protein transduction:

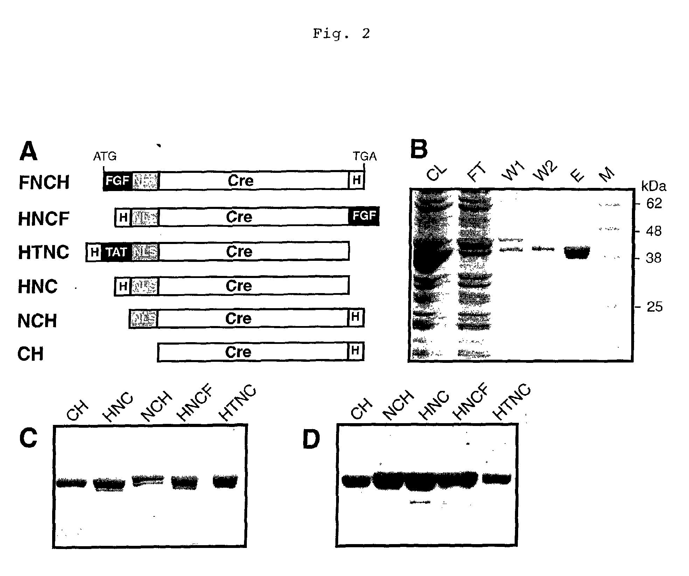

Protein transduction is a recently developed method to introduce biologically active proteins directly into mammalian cells with high efficiency (Fig. 2; Hawiger, J. Curr. Opin. Chem. Biol. 3, 89-94 (1999); Schwarze, S.R. et al., Tr. Cell Biol. 10, 290-295 (2000)). Here, transcription and translation is completely separated from the host cell ideally carried out in a bacterial expression system. Recombinant technology has been used to modify biophysical properties of proteins particularly with respect to their cell permeability by employing so-called protein transduction domains (PTDs) (Nagahara, H. et al., Nat. Med. 4, 1449-1452 (1998); Schwarze, S.R. et al., Science 285, 1569-1572 (1999); Rojas, M. et al., Nat. Biotechnol. 16, 370-375 (1998)).
It has been demonstrated that a basic 11 amino acid (aa) peptide, derived from HIV TAT, renders β-galactosidase (β-gal) into a cell-permeable form. β-gal activity can be detected in organs such as liver, kidney, lung, brain and spleen of mice after intraperitoneal injection of TAT-β-gal (Schwarze, S.R. et al., Science 285, 1569-1572 (1999)).
Besides the basic TAT peptide also other peptides or protein (fragments) have been reported to enhance cellular uptake of proteins (see table 1), such as a hydrophobic peptide modified from Kaposi fibroblast growth factor (FGF) (Rojas, M. et al., Nat. Biotechnol. 16, 370-375 (1998); Lin, Y.Z. et al., J. Biol. Chem. 270, 14255-14258 (1995)).
Recently it has been reported that a fusion protein of NLS, Cre recombinase and a FGF peptide displays cell permeability. After direct application of the recombinant protein to cultured cells of a T lymphocyte line containing a loxP-modified substrate Cre-mediated recombination was observed in approx. 80 % of the cells (Jo, D. et al., Nat. Biotechnol. 19, 929-933 (2001)). However, it remainded unclear from this study whether indeed the FGF peptide is essential for transduction or even contributes significantly to the cellular uptake of Cre, since no direct comparison with a corresponding protein lacking the FGF peptide has been reported.

### Functional role of the nuclear localization signal (NLS) for Cre activity:

Irrespective of the mode of delivery, either by transfection, infection, transduction or infection, the Cre protein has to enter the nucleus for efficient recombination of loxP-target-sequences. Initially it has been hypothesized that Cre enters the nucleus by passive diffusion through the nuclear pore (Sauer, Mol. Cell. Biol. 7, 2087-2096 (1987)). The Cre protein is 38 kDa in size and therefore smaller than the approx. 50 kDa upper size limit of the nuclear pore (Peters, J. Biol. Chem. 258, 11427-11429 (1983)). However, most nuclear proteins of eukaryotes, irrespective of the size, carry a specific NLS which enhances the nuclear translocation (Dingwall & Laskey, Tr. Biochem. Sci. 16, 478-481 (1991)). Consequently, addition of a NLS to proteins usually results in increased nuclear concentration (Breeuwer & Goldfarb, Cell 60, 999-1008 (1990)).

Nuclear localization sequences (NLSs) fall into three classes. Two of these are highly basic in nature, those displaying homology to the well-characterized SV40 large T antigen of basic amino acids (PKKKRKV; SEQ ID NO:5) and bipartite NLSs which contain two stretches of basic amino acids separated by a spacer of 10-12 aa (Robbins et al., Cell 64 (1991) 615-623), e.g. that of nucleoplasmin (KRpaatkkagqaKKKK; SEQ ID NO:42). The third class of NLSs include those resembling the yeast homeodomain containing protein Matα2 (Hall et al., PNAS 87, 6954-6958 (1990)) or the protooncogene c-myc (Makkerh et al., Curr. Biol. 6 (1996) 1025-1027) where no particular accumulation of basic aa occurs. All classes of NLS are believed to be recognized specifically by the α/β1-importin heterodimer, as demonstrated for many proteins from several species (see overview in Jans et al. Bioessays 22, 532-544 (2000)). In order to increase the recombination efficiency NLS has been fused to the Cre recombinase (Gu et al., Cell 73, 1155-1164 (1993)). Indeed, recombination efficiency has been reported to increase approx. 2-fold after transfection of a NLS-Cre encoding construct as compared to a construct lacking NLS. This result indicated that the NLS can improve the nuclear translocation. However, more recently it has been demonstrated by a comprehensive quantitative analysis that addition of a NLS does not enhance nuclear translocation of Cre recombinase (Le et al., Nuc. Acids Res. 27, 4703-4709 (1999)). Moreover it has been reported that wild-type Cre efficiently enters the nucleus presumably due to a endogenous bipartite-like NLS within Cre. Thus, the addition of an exogenous NLS to Cre seems not to enhance Cre-mediated recombination (Le et al., Nuc. Acids Res. 27, 4703-4709 (1999)).

### Summary of the Invention

It was now found that a particular modified nuclear localization signal (NLS) - if fused to a recombinase - significantly enhances the cellular uptake of the recombinases and subsequent recombination. Namely, the FGF peptide has unexpectedly found to be not essential for transduction, whereas the modified SV40-NLS alone has found to confer cellular uptake of the Cre recombinase, resulting in efficient Cre-mediated recombination of the loxPo-modified target sequences . In the modified NLS the first proline residue of the SV40-NLS is deleted, since proline confers unique conformational constraints. The presence of a proline within a polypeptide disturbs secondary structure elements, such as α-helices (Vanhoof et al., FASEB J. 9, 736-744 (1995)), which might be deleterious for the enzymatic activity and/or transduction of the Cre fusion protein. The deletion of the proline residue unexpectedly resulted in an improved enzymatic activity and/or an improved transduction efficiency.

The effect is further enhanced if the fusion protein further comprises a protein transduction domain, such as a TAT transduction domain. In particular, the inventors found that Cre transduction is strictly dependent on time and concentration but is independent of the transduction peptide FGF, whereas fusion with the TAT peptide significantly enhances cellular uptake. More than 95 % recombination efficiency in fibroblast cells as well as murine embryonic stem cells can be achieved after His-TAT-NLS-Cre (HTNC) transduction. Efficient recombination can also be achieved in primary splenocytes ex *vivo.* It is expected that application of said fusion protein, which can be produced readily in large quantities from a bacterial source, will expand the abilities to manipulate mammalian genomes. The present invention thus provides:
(1) a fusion protein comprising
   (a) a site-specific DNA recombinase domain,
   (b) a domain containing a modified nuclear localization signal (NLS) of type one or two which differs from a natural, Pro-containing NLS in that it lacks the Pro residue(s);
(2) in a preferred embodiment of (1) the fusion protein further comprises a protein transduction domain;
(3) the use of a fusion protein as defined in (1) and (2) above for preparing an agent for inducing target gene alterations in living organisms or in cell cultures, wherein said living organisms or cells of said cell cultures carry at least one or more recognition sites for said site-specific DNA recombinase integrated in its genome;
(4) the use of a fusion protein comprising
   (a) a site specific DNA recombinase domain
   (b') a domain containing a nuclear localisation signal (NLS) of type one, two or three
   for preparing an agent for inducing target gene alterations in living organisms or cell cultures, wherein said living organisms or in cells of said cell cultures carry at least one or more recognition sites for said site-specific DNA recombinase integrated in its genome;
(5) a method for inducing gene alterations in living organisms or cell cultures which comprises administering to said living organisms or to cells of said cell cultures, a fusion protein as defined in (1) and (2) above, wherein said living organism carries at least one or more recognition sites for said site-specific DNA recombinase integrated in its genome;
(6) a method for inducing gene alterations in living organisms or cell cultures, which method comprises administering to said living organisms or to cells of said cell cultures a fusion protein as defined in (4) above, wherein said living organisms carry at least one or more recognition sites for said site-specific DNA recombinase integrated in its genome;
(7) a DNA sequence coding for the fusion protein as defined in (1) and (2) above;
(8) a vector comprising the DNA sequence of (7) above;
(9) a host cell transformed with the vector of (8) above and/or comprising the DNA of (7) above;
(10) a transgene organism carrying the DNA as defined in (7) above in its genome;
(11) a method for producing the fusion protein of (1) and (2) above which comprises culturing the transformed host cell of (9) above and isolating the fusion protein;
(12) an injectable composition comprising the fusion protein as defined in (1) and (2) above;
(13) the method for inducing gene alterations in a living organisms or cell cultures which comprises
   (i) transfection or viral infection of living organisms or cell cultures with a DNA as defined in (7) above or a vector as defined in (8) above, or
   (ii) preparing transgene living organisms or cell cultures containing a DNA sequence as defined in (7) above integrated in its genome, wherein said living organisms or cells of said cell culture carry at least one or more recognition sites for said site-specific DNA recombinase integrated in its genome;
(14) the use of a modified localization signal (NLS) as defined in (1) above for enhancing cellular uptake of functional biopolymers in living organisms or cell cultures.

### Short Description of the Figures

Fig. 1: Alternative approaches to deliver Cre recombinase to living cells (rCre := recombinant Cre)
Fig. 2: Design of expression cassettes and purification of Cre fusion proteins. (A) Utilized expression construct. (B) SDS-PAGE analysis of the purification employing Ni(II)-affinity chromatography. (C) Comparison of purified fusion proteins on a coomassie stained gel. (D) Immunoblot analysis of purified Cre fusion proteins employing an anti-Cre antibody.
Fig. 3: Transduction of Cre-fusion proteins into a fibroblast Cre reporter cell line. (A) Schematic representation of the reporter gene construct of the fibroblast reporter cell line CV1-5B. (B) Incubation of CV1-5B cells for 16 h in medium containing 2.5 µM purified Cre fusion protein. (C) Quantification of β-gal activities of reporter cells incubated for 16 h with various concentrations of Cre fusion proteins. (D,E) Southern blot analysis of transduction and subsequent recombination. (D) Time kinetics of HNCF and HNC transduction. (E) Concentration dependency of HNCF and HNC transduction.
Fig. 4: (A) Comparison of Cre recombinase activities of HNCF, NCH and CH using a cell-free activity assay. (B) Quantitative analysis of recombinase activities of NCH and CH after transfection. (C) Comparison of the time dependency of HNC- and HTNC-transduction.
Fig. 5: Detection of Cre in cells after transduction employing an anti-Cre antibody.
Fig. 6: HTNC transduction into (A) murine embryonic stem cells and (B,C) splenocytes *ex vivo* analyzed by Southern blot.

### Detailed Description of the Invention

The following abbreviations are used: aa, amino acid(s); β-gal, β-galactosidase; ES, embryonic stem cell(s); FGF, fibroblast growth factor; NLS, nuclear localization signal; PTD, protein transduction domain; RRS, recombinase recognition site.

The expression "target sequences" according to the present invention means all kind of sequences which may be mutated (viz. deleted, translocated, integrated and/or inverted) by the action of the recombinase. The number of RRS in the target sequence depends on the kind of mutation to be performed by the recombinase. For most of the mutations (especially for deletions and invertions) two RRS are required which are flanking the sequence to be mutated (deleted or inverted). For some kinds of integrations only one RRS may be necessary within the target sequence.

The "living organisms" according to the present invention are multi-cell organisms and can be vertebrates such as mammals (e.g., humans and non-human mammals including, but not limited to, rodents such as mice or rats) or non-mammals (e.g., fish) or can be invertebrates such as insects or worms, or can be plants (higher plants, algi or fungi); or are microorganisms such as yeasts. Most preferred living organisms are mice and fish.

"Cell culture" according to the present invention include cells isolated from the above defined living organism (including humans and non-human mammals) and cells of the microorganism and cultured *in vitro.* The above cells derived from multi-cell organisms can be transformed (immortalized) or untransformed, i.e. directly derived from the living organism; primary cell culture or cell lines (e.g. embryonic stem cells or embryonic germ cells).

The site-specific DNA recombinase domain within the fusion protein of embodiments (1), (2), (4) and (6) is preferably selected from a recombinase protein derived from Cre, Flp, φC31 recombinase (Thorpe and Smith, Proc. Natl. Acad. Sci, USA, vol. 95, 5505-5510 (1998)), γδ resolvase (Schwickardi and Dröge, FEBS letters 471:147-150 (2000) and R recombinase (Araki et al., J. Mol. Biol., 182, 191-203 (1985)). The preferred recombinases are Cre and mutants thereof (preferably the Cre variant of aa 15 to 357 of SEQ ID NO:2 and Flp and variants thereof including Flpe (preferably the Flp variant of aa 15 to 437 of SEQ ID NO:4.

The modified NLS according to embodiments (1), (2) and (14) is a type one or two NLS which is a highly basic peptide and which is preferably derived from a natural SV40 NLS of type one or from a homologue thereof. Natural SV40 NLS of type one having a Pro-residue include, but are not limited to, PKKRKV (SEQ ID NO:5), APTKRKGS (SEQ ID NO:52) and PNKKKRK (SEQ ID NO:53). The natural NLS are modified so as to lack the proline residue at its N-terminus (as in case of SEQ ID NO:5 to yield the peptide of SEQ ID NO:6) or to lack the Pro within the sequence (as in case of SEQ ID NO:52). It is also contemplated that by the modification of one or two amino acid residues flanking the Pro residue are deleted together with the pro-residue (e.g. as in SEQ ID NO:52 "AP" or "APT" might be deleted). The term "derived from a natural SV40 NLS of type one" also includes NLS displaying homology with said modified SV40 NLS and preferably refers to a NLS peptide having five to ten amino acid residues and containing at least five basic amino acid residues (such as Arg, Lys and the like) and no Pro residue. Examples of the modified NLS include, but are not limited to, KKKRKV (SEQ ID NO:6), KKKKKV (SEQ ID NO:45), KKKKKK (SEQ ID NO:46), KKRRKV (SEQ ID NO:47), KRRRKV (SEQ ID NO:48), RRRRKV (SEQ ID NO:49), RRRRRV (SEQ ID NO:50), RRRRRR (SEQ ID NO:51) and NKKKRK (SEQ ID NO:54). The preferred type one NLS according to the present invention has the sequence KKKRKV (SEQ ID NO:6). The type two NLS (also called bipartitive NLS) is derived from the type one NLS as defined hereinbefore in that the basic amino acid sequence of the type one NLS is separated into two particular sequences connected by an arbitrary spacer of 10-12 amino acids, such as KK-spacer-KKKK, RR-spacer-RRRR, RR-spacer KKKK, KK-spacer-RRRR, RR-spacer-RKKK, RR-spacer-RRKK, RR-spacer-RRRK, RR-spacer RRRR and derivatives thereof. Preferred NLS of type two is KR-spacer-KKKK.

In embodiment (1) of the invention the fusion protein domains (a) and (b) may be directly or through a linker peptide linked to each other. With regard to suitable linker peptides see below. Moreover, the domain (b) may be linked to the N- or C-terminal of the domain (a). It is, however, preferred that the domain (b) is linked to the N-terminal of the domain (a). A particularly preferred fusion protein has the amino acid sequence shown in SEQ ID NO:37 or 39.

According to embodiment (2) of the invention the fusion protein further contains (c) a protein transduction domain (PTD). The PTD according to the present invention includes, but is not limited to, the PTDs mentioned in the passage Background of the Invention. Suitable PTDs are summarized in Table 1 below.

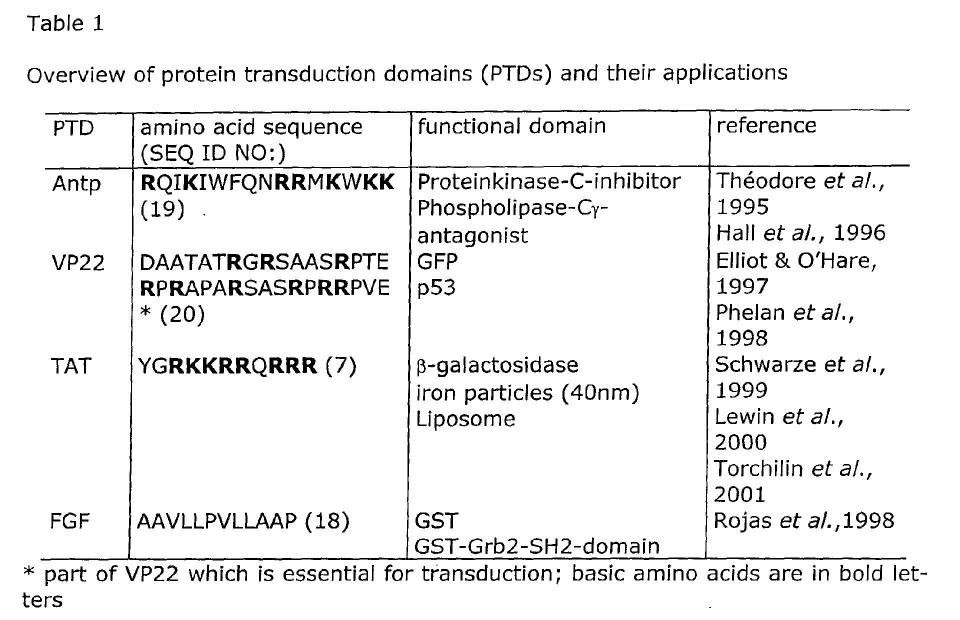

The PTD preferably is derived from the VP22 protein of HSV or from the TAT protein of HIV. Suitable TAT proteins include, but are not limited to, proteins comprising the amino acid sequence shown in SEQ ID NO:7 and mutant thereof such as proteins comprising the amino acid GRKKRRQRRR (SEQ ID NO:8) AGRKKRRQRRR (SEQ ID NO:9) YARKARRQARR (SEQ ID NO:10) YARAAARQARA (SEQ ID NO:11) YARAARRAARR (SEQ ID NO:12) YARAARRAARA (SEQ ID NO:13) YARRRRRRRRR (SEQ ID NO:14) and

YAAARRRRRR (SEQ ID NO:15).

Preferred are transduction domains consisting of the TAT proteins having one of the SEQ ID NOs depicted above.

Suitable VP22 proteins include, but are not limited to, the wild-type VP22 protein, i.e., a protein comprising amino acids 1 to 302 of SEQ ID No:16, and truncated forms thereof. Truncated VP22 proteins in accordance with the present invention can be those lacking 1 to 158 amino acid residues at their N-terminal end. The most preferred VP22 protein is the truncated VP22 PTD shown in SEQ ID NO:17.

A suitable FGF protein has the sequence AAVLLPVLLAAP (SEQ ID NO:18).

The domain (c) can be fused to domains (a) and (b) at any possible position, i.e., the protein transduction domain can be fused to the N- or C-terminal of the site-specific DNA recombinase and NLS or can be fused to active sites within the fusion proteins out of site-specific DNA recombinase or between domains (a) and (b). Preferably the protein transfusion domain is fused to the N-terminal of a fusion protein out of the two domains (a) and (b) having the domain (b) at its N-terminal (viz. a fusion protein N-PTD-NLS-recombinase).

The protein transduction domain can be fused to the site-specific DNA recombinase and the NLS either through a direct chemical bond or through a linker molecule. Such linker molecule can be any bivalent chemical structure capable of linking the two domains. The preferred linker molecule according to the present invention is a short peptide, e.g., having 1 to 20, preferably 1 to 10, amino acid residues. Specifically preferred short peptides are essentially consisting of Gly, Ala and/or Leu.

The fusion protein of embodiment (1) or (2) of the present application may further comprise other functional sequences (d) (differing from the functional sequences of domains (b) and (c), i.e. non-NLS and non-PTD sequences) such as secretion conferring signals, signals conferring protein stabilisation, signals facilitating purification (such as His-tags) and the like.

A preferred fusion protein with domains (a) to (c) is that of SEQ ID NO:1.

According to embodiment (7), the invention also relates to DNA sequences encoding the fusion proteins of embodiments (1) and (2). It is to be understood that any DNA sequence and derivative is included as long as it encodes the fusion protein.

In case the fusion protein comprises a protein transduction domain derived from the TAT protein of HIV, the DNA sequence coding for said fusion protein preferably comprises the sequence

5′ TAC GGC CGC AAG AAG CGC CGC CAA CGC CGC CGC 3′ (SEQ ID NO:21).

Such a preferred DNA sequence is for instance shown in SEQ ID NO:43. In said sequence the 3' terminal codon ggc codes for the linker Gly. The DNA sequence of a suitable recombinase may be directly attached to said codon ggc.

The fusion protein of embodiments (1) and (2) can be obtained by the method of embodiment (11), said method may preferably comprise the following steps:
1. Fusion of the recombinase coding region (e.g. encoding Cre: see SEQ ID NO: 2) with the NLS sequence (e.g. a sequence encoding the modified NLS of SEQ ID NO:6) and optionally with the sequence conferring protein translocation (e.g. the sequence encoding the TAT peptide YGRKKRRQRRR, SEQ ID NO:7) using standard cloning protocols (Maniatis et al., Cold Spring Harbor Laboratory, New York (1989)) or chemical synthesis.
2. Generation of a construct for the expression of the fusion protein in prokaryotic or eukaryotic cells, e.g. in E. coli DH5a (Hanahan, J. Mol. Biol.;166(4):557-80 (1983)) using the QIAexpress pQE vector (Qiagen, Hilden).
3. Expression of the above mentioned fusion protein in prokaryotic or eukaryotic cells, e.g. in E. coli DH5a (Hanahan, 1983)
4. Extraction and purification of the above mentioned fusion protein e.g. as described in Nagahara et al., Nat. Med., 4(12):1449-52 (1998).

The use of embodiments (3), (4), (13) and (14) and the method of embodiments (5) and (6) can be performed according to the skills of an artisan in the light of the art cited in the passage "Background Art".

The fusion protein of embodiments (4) and (6) is free of a PTD sequence (domain) as defined hereinbefore. As site specific DNA recombinase domain (a) any DNA recombinase mentioned hereinbefore can be utilized. Domain (b') may contain any naturally occurring NLS of type one, two or three mentioned in the passage "Background Art" especially those mentioned in the review of Jans et al., Bioassays 22, 532-544 (2000)), which is herewith incorporated by reference. Preferred is the NLS sequence of SEQ ID NO:5. It is moreover preferred that domain (b') is linked to the N-terminal of domain (a).

In embodiment (14) any modified NLS mentioned hereinbefore can be utilized. In accordance with the invention, said modified NLS is coupled to the functional biopolymer (i.e. directly or through a linker, e.g. a linker as defined hereinbefore). Suitable functional biopolymers include, but are not limited to, proteins such as
- enzymes, including, but not limited to recombinases, kinases and phosphatases
- Marker proteins, including, but not limited to fluorescent proteins, luciferase, β-galactosidase
- regulatory Peptides, for inhibition or activation of other proteins
- Transcription factors
nucleic acids such as
- antisense nucleotides (e.g. phosphoamidate-morpholino-oligonucleotides "PMOs")
- expression plasmids (e.g. for expression of marker proteins or transcription factors).

The present invention also relates to the constructs out of modified NLS and functional biopolymers as defined above.

The invention is further explained by the following examples which are however, not to be construed as to limit the invention. In said examples the generation of expression vectors encoding Cre recombinase fused to the particular NLS of SEQ ID NO:6 and to FGF and TAT peptides, respectively, is disclosed. The potentials of the recombinant proteins to transduce and subsequently recombine loxP-flanked targets in mammalian cells were compared side by side with Cre lacking any particular PTD.

### Examples

### Materials and Methods

### Plasmid constructions:

A TAT-encoding fragment was generated by polymerase chain reaction (PCR) using primers NPTatU and NeuUS. This fragment was cloned into the pTriEx-1 vector (Novagen) via NcoI and XhoI restriction sites, resulting in pTriEx-TAT-U-H. This vector encodes in addition to TAT a second protein fragment designated as 'U' which was not used in this study. The corresponding DNA-fragment was deleted by digestion with SpeI and subsequent religation, resulting in pTriEx-TAT-H. The TAT-encoding fragment was removed resulting in pTriEx-H by PstI digestion. To obtain pTriEx-CH a Cre encoding PCR product was generated from the template pPGK-Cre-bpA (Wunderlich, F.T. et al., Nucleic Acids Res. 29, e47 (2001)) using primers 5H3cre and 3X1cre. This fragment was subsequently cloned into the pTriEx-H using HindIII and XhoI sites. To generate pTriEx-NCH a NLS-Cre encoding fragment was amplified using primers 5H3NLScre and 3X1cre and cloned as above. To construct pTriEx-FNCH annealed oligonucleotides 5PstFGF and 3PstFGF were cloned into the PstI restriction site of pTriEx-NCH. To generate pTriEx-HTNC a NLS-Cre-STOP encoding PCR product was generated using primers 5H3NLScre and 3creStopXho and subsequently cloned into pTriEx-TAT-H via HindIII and Xho restriction sites; the His-tag was introduced by cloning annealed oligonucleotides NcoHis5 and NcoHis3 into the NcoI site. pTriEx-HNCF was constructed by cloning annealed oligonucleotides NcoHis5 and NcoHis3 as well as 5XhoFGF and 3XhoFGF into the NcoI and XhoI restriction sites, respectively, of pTriEx-NCH. From pTriEx-HTNC a 700 bp fragment was prepared using BamHI / XhoI restricition sites and cloned into pTriEx-HNCF, resulting in pTriEx-HNG. All PCR-generated fragments were confirmed by sequencing.

Primers (SEQ ID NO):
NPTatU: (5'-CTTGGCCATGGGCGCTGCAGGTCGCAAGAAACGTCGCCAACGTCGCCGTC
CGCCTGCAGG CACTAGTCAGAT TTTCGTCAAG ACTTTGAC-3') (22)
NeuUS: (5'-CCATTACTAGTTGCGCCCATACCACCACGTAGCCTTAGCACAAGATG-3')
(23)
5H3cre: (5'-CGTCCAAGCTTGTCCAATTTACTGACCGTACACC-3') (24)
3X1cre: (5'-CTGAACTCGAGACCATCGCCATCTTCCAGCAGGC-3') (25)
5H3NLScre: (5'-CATGGAAGCTTGAAGAAGAGGAAGGTGTC-3') (26)
5PstFGF: (5'-GTAGCTTTACTTCCGGCAGTTCTGTTAGCGTTGCTGGCACCGGGTGCA-3')
(27)
3PstFGF: (5'-CCCGGTGCCAGCAACGCTAACAGAACTGCCGGAAGTAAAGCTACTGCA-3')
(28)
3creStopXho: (5'-CTAATCTCGAGCTAATCGCCATCTTCCAGCAG-3') (29)
NcoHis5: (5'-CATGGGCCATCACCATCACCATCACGG-3') (30)
NcoHis3: (5'-CATGCCGTGATGGTGATGGTGATGGCC-3') (31)
5XhoFGF: (5'-TCGAGGGTGCAGCTGTATTACTTCCGGTTCTGTTAGCGGCACCGTGAT-3')
(32)
3XhoFGF: (5'-TCGAATCACGGTGCCGCTAACAGAACCGGAAGTAATACAGCTGCACCC-3')
(33).

### Expression and purification of recombinant proteins:

pTriEx plasmids were used to transform E. coli strain TUNER (DE3)pLacI (Novagen) allowing IPTG inducible expression of His-tagged proteins. An enriched medium of LB + 1 % Glucose containing 50 µg/ml carbenicillin and 34 µg/ml chloramphenicol was used to inoculate overnight cultures. Usually 500 ml cultures of LB containing 100 µg/ml ampicillin and 34 µg/ml chloramphenicol were inoculated 1:50 and grown at 37° C until an OD600 of 0.7. Over-expression was induced by adding IPTG to a final concentration of 0.5 mM and an additional incubation of 3-4 hours at 37° C. Cells were harvested by centrifugation and frozen in dry ice/ethanol and stored at -20°C. Cell pellets were thawed by resuspending in lysis buffer (100 mM NaH₂PO₄, 10 mM Tris, pH 8.0, 300 mM NaCl, 10 mM imidazole) at 5 ml per gram wet weight. Cleared lysate was obtained after centrifugation for 25 min at 16.000 rpm at 4°C. 1 ml of 50% Ni-NTA slurry (Qiagen) was added to 5 ml cleared lysate and mixed gently by shaking at 4°C for 1 h. The slurry was packed into a column and washed with 10 bed volumes (100 mM NaH₂PO₄, 10 mM Tris, pH 8.0, 300 mM NaCl, 20 mM imidazole). Recombinant protein was eluted (100 mM NaH₂PO₄, 10 mM Tris, pH 8.0, 300 mM NaCl, 250 mM imidazole) and either dialyzed against appropriate media (see below) for immediate use or frozen at -20° C for storage up to 8 weeks without significant loss of activity. Protein concentrations were measured using Bradford reagent (Sigma) and/or Warburg method. Cre activities were determined by a cell-free assay as described (Jo, D. et al., Nat. Biotechnol. 19, 929-933 (2001); Wunderlich, F.T. et al., Nucleic Acids Res. 29, e47 (2001)) except using 250 ng of substrate DNA (Cantor, E.J., Chong., S., Protein Expr. Purif. 22, 135-140 (2001)). For quantification reactions were transformed into E. coli and colonies counted. A commercially available Cre protein (New England Biolabs) was used as a standard.

### SDS-PAGE and immunoblot analysis:

Samples were separated by SDS-PAGE (10 %), transferred to nitrocellulose membranes (Amersham) and probed with anti-Cre (Novagen) or anti-Penta-His (Qiagen) antiserum. Anti-Cre was incubated 1:5000 in PBS containing 5 % dry milk, anti-Penta-His was used 1:2000 in PBS containing 3 % BSA. Blocking was carried out in corresponding buffers without antibody for 1 hour at room temperature. As secondary antibodies anti-rabbit-POD (Vector) or anti-mouse-POD conjugates (Amersham) were used both at a 1:10000 dilution in PBS containing 5 % milk powder. Blots were washed using PBST (PBS containing 0.5 % Tween 20). Detection was carried out using chemiluminescence (ECL, Amersham).

### Cell culture, transfection of and transduction into CV fibroblasts:

CV1-5B (Kellendonk, C. et al., Nucleic Acid Res. 24, 1404-1411 (1996)) cells were cultured in Dulbecco's modified Eagles's medium (DMEM) containing 10% fetal calf serum and 100 U/ml penicillin and 0.1 mg/ml streptomycin. Transfection experiments were carried out in 24-well plates using 1 mg plasmid as described previously (32). For Cre transduction experiments 2 x 10⁶ cells were plated on a 24-well-plate, grown for 24 h or until 90 % confluency. Cre protein was diaylized against a 1:1 mixture of DMEM and PBS containing 0.1 % pluronic F-68 (Sigma) and sterilized by filtration using a 0.2 mm filter disk (Millipore). Cells were incubated with Cre containing medium at indicated concentrations for indicated times. After transduction cells were washed using PBS and cultured 60 h in normal medium. For determination of b-gal activity cells were fixed and stained as described previously (Kellendonk, C. et al., Nucleic Acid Res. 24, 1404-1411 (1996)). For Southern blot analysis genomic DNA was extracted from cells by proteinase K digestion and isopropanol precipitation. DNA was digested overnight using EcoRI restriction enzyme and separated on an agarose gel. DNA was immobilized on a Hybond nitrocellulose membran (Amersham) and probed with a ³²P-labelled LacZ fragment. Quantification of bands was carried out by phosphor imaging technique (FUJIX BAS 1000; TINA 2.09 software).

### Immunofluorescence:

CV1-5B Fibroblasts were grown on 24-well tissue culture plates until 90 % confluency. After a 4 h incubation in Cre fusion protein containing DMEM/PBS (1:1) cells were washed twice with PBS, trypsinized and plated on cover-slips. After 16 h in culture cells were fixed with cold methanol (-20°C). The samples were washed three times with PBS and blocked for 30 min. Blocking and antibody incubations were carried out in PBS containing 1% dry milk. Antibody detection was performed using polyclonal rabbit anti-Cre antibody (Babco, 1:1000 dilution; 90 minutes) and FITC-anti-rabbit IgG (Sigma, 1:50 dilution; 60 minutes). The nuclei were stained by adding 0,01 mg/ml 4,6-di-amino-2-phenylindol (DAPI) in PBS before mounting on glass slides. Cells were observed in an epifluorescence microscope (Zeiss Axiophot) and documented on color slide film. For reproduction images were scanned directly from slides.

### Cre transduction into mouse embryonic stem cells:

ES cell culture was carried out as described elsewhere (Torres, R.M. & Kühn, R., Laboratory protocols for conditional gene targeting, Oxford University Press, Oxford (1997)). Single cell suspensions of subconfluent plates were prepared by trypsinization. Cells were washed in PBS and resuspended in Cre containing medium. Cre protein was diaylized against a 1:1 mixture of DMEM and PBS and sterilized by filtration. After resuspension in Cre containing medium ES cells were plated on appropriate plates containing mitomycin C treated embryonic feeder cells. In a typical experiment 2.5 x 10⁵ cells were used in a volume of 0.5 ml plated in a well of a 24-well plate. After transduction cells were washed and cultured at least three days in normal ES-medium. Genomic DNA was extracted and used for Southern blot analysis as described above, except using EcoRV digestion and a ROSA26 specific probe.

### Ex vivo Cre transduction into mouse splenocytes:

For transduction into primary splenocytes we used polb-flox (Gu, H. et al., Science 265, 103-106 (1994)) mice. Freshly prepared splenic cells were washed in PBS and resuspended in Cre containing medium. Cre protein was diaylized first against PBS, then against protein-free medium (Hyclone), and sterilized by filtration. 5 x 10⁶ cells were resuspended in 1 ml Cre containing medium and plated in a well of a 24-well plate. After transduction cells were harvested, resuspended in normal B cell medium and plated in a well of a 24-well plate. After incubation for 16 hours genomic DNA was extracted and used for Southern blot analysis as described above, except using BamHI digestion and a polb specific probe (Gu, H. et al., Science 265, 103-106 (1994)). Splenocytes were stained with fluorochrome-conjugated monoclonal antibodies (fluorescein-isothiocyanate, FITC; phycoerythrin, PE). Antibodies against CD19 (Pharmingen) and Thy1.2(CFO1) (in house) were used. For determination of cell viability propidium iodide (PI) staining was performed. FACS analysis was carried out using a FACScan (Becton Dickinson); for sorting a FACStar+ was used (Becton Dickinson).

### Results and Discussion:

In order to evaluate the potency of the PTDs FGF and TAT to promote cellular uptake of Cre recombinase into mammalian cells we generated expression vectors encoding FNCH (FGF-NLS-Cre-His), HNCF (His-NLS-Cre-FGF) as well as HTNC (His-TAT-NLS-Cre; SEQ ID NO:40) (Fig. 2A). All constructs include a His-tag for single-step purification of recombinant proteins from a bacterial source. As controls we also generated Cre expression constructs lacking any particular transduction domain, but carrying the His-tag either at the N-terminus (His-NLS-Cre, abbreviated HNC; SEQ ID NO:38) or C-terminus (NLS-Cre-His, NCH) of Cre.

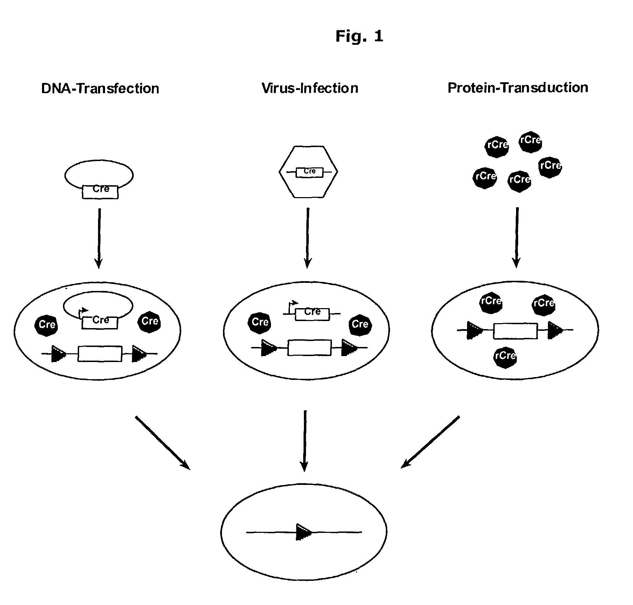

Finally we generated the expression construct Cre-His (CH; SEQ ID NO:34) which lacks NLS and encodes a C-terminally His-tagged Cre recombinase (Fig. 2A). Analysis of expression revealed that one recombinant protein, namely FNCH, displayed poor solubility under native conditions (data not shown) whereas the other recombinant proteins could be highly enriched under native conditions (Fig. 2B,C). HTNC displayed reproducibly the highest level of expression (approx. 30 mg/l culture), whereas the other proteins showed expression levels of about 10 mg/l. All tested proteins were detected by antibodies directed against Cre (Fig. 1D) and the His-tag (data not shown), respectively, and displayed the expected size of approx. 41 kDa. All proteins, except HTNC, are soluble in physiological buffers such as PBS up to a concentration of approx. 12 mM (0.48 mg/ml). HTNC can be concentrated up to 16 mM (0.64 mg/ml).

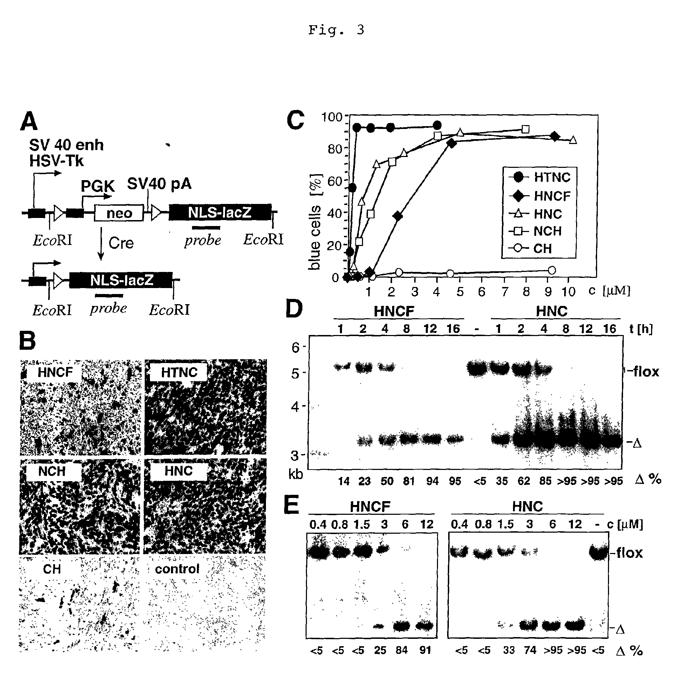

In order to assess the capacity of recombinant Cre fusion proteins to invade eucaryotic cells and subsequently perform recombination of a loxP-flanked substrate, we used the well established reporter cell line CV1-5B (Kellendonk et al., Nucleic Acids Res. 24, 1404-1411 (1996)). This fibroblast cell line contains a single copy of a stably integrated reporter construct. Cre activity is monitored by deletion of a loxP-flanked stop segment resulting in the activation of a LacZ gene (Fig. 3A). Reporter cells were incubated 16 h with medium containing 2.5 mM (approx. 100 mg/ml) of HNCF, HNC, NCH, CH and HTNC. Indeed, b-gal activity was detected in cells incubated with fusion proteins containing a PTD, namely HNCF and HTNC, indicative of cellular uptake and subsequent recombination of the reporter gene. Unexpectedly, cells incubated with HNCF displayed only a low percentage of blue cells, whereas incubation of the control proteins lacking the PTD, namely HNC and NCH resulted in a significantly higher percentage of stained cells (Fig. 3B). A dose-response analysis revealed that transduction of HNCF, HNC as well as NCH and subsequent recombination is strictly dependent on concentration and reaches a level of saturation of more than 90 % efficiency at approx. 5 mM (Fig. 3C). Half-maximal recombination is achieved for NCH and HNC between 1 and 2 mM, whereas approx. 3 to 4 mM HNCF are needed for the same level of recombination. In order to demonstrate Cre-mediated recombination not only by activation of a reporter gene but also at DNA level, we performed Southern blot analysis of Cre-transduced CV1-5B reporter cells. We compared HNCF side-by-side with HNC to directly assess the function of the FGF peptide. A time course experiment revealed that incubation of the cells with 12 mM HNCF for 1 hour results in about 15 % recombination efficiency. Half-maximum recombination is achieved after 4 hours of incubation (Fig. 3D). Application of 12 mM HNC protein is more efficient; half-maximal recombination is achieved already after 1 to 2 hours and 8 hours are sufficient to perform recombination in more than 95 % of the cells. In order to assess the concentration dependency of transduction, we incubated reporter cells in serial dilutions of HNCF and HNC down to 0.4 mM. Southern blot analysis demonstrated that applications of up to 0.8 mM of both either HNCF or HNC did not result in any detectable recombination (Fig. 3E). However, application of 1.5 mM HNC resulted in substantial recombination (∼ 30 %) whereas at the same concentration HNCF is almost inactive (∼ 2 %). Recombination in almost every cell is achieved by using 6 mM HNC and 12 mM HNCF, respectively (Fig. 3E). Recently it has been reported that a cell permeable Cre, namely His-NLS-Cre-MTS which is almost identical to the HNCF protein used in our study, can enter eucaryotic cells and perform recombination (Jo, D. et al., Nat. Biotechnol. 19, 929-933 (2001)). However, the actual ability of FGF to promote this process was not investigated. Our comparative analysis of HNC and HNCF demonstrated that transduction of His-NLS-Cre and subsequent recombination is independent of the hydrophobic FGF peptide. Therefore the FGF peptide is dipensable for efficient transduction of His-NLS-Cre. Moreover, the analysis of the time and concentration dependency of HNCF as compared to HNC demonstrated that application of a NLS-Cre fusion protein lacking the transduction peptide FGF is more effective.

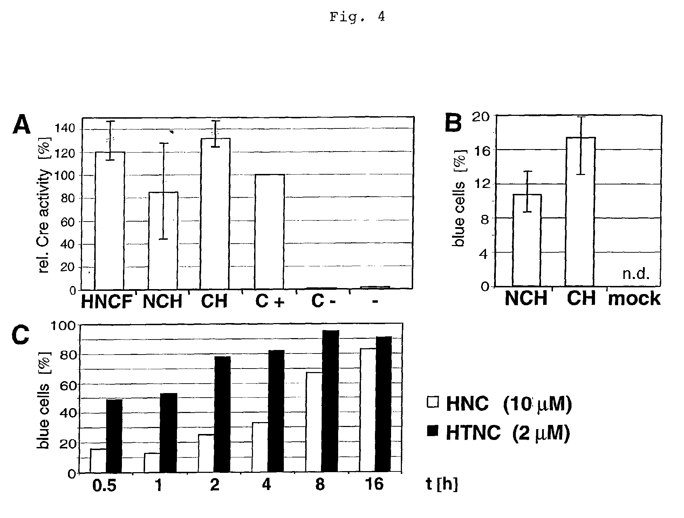

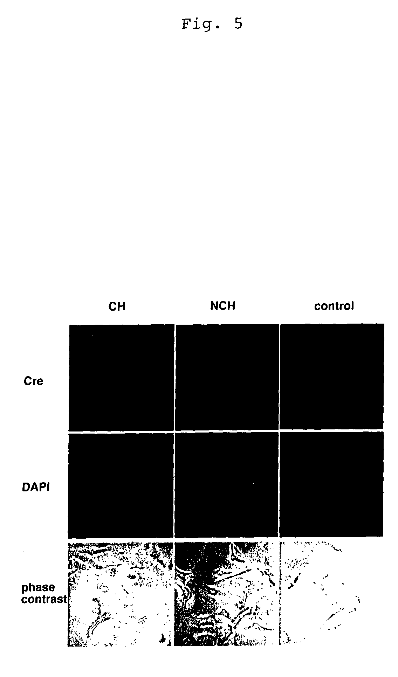

All recombinant Cre fusion proteins analysed in this study resulted in highly efficient transduction and subsequent recombination, with one exception, namely CH (Fig. 3B). Recombination efficiency after transduction using CH did not increase over 5 % whereas NCH and HNC, the corresponding constructs carrying a NLS, resulted in efficiencies of more than 90 % (Fig. 3C). There are three possible explanations for this observation: i) the enzymatic activity of HNC and NCH, respectively, is higher than that of CH; this is unlikely because CH and NCH displayed comparable specific activities in a cell-free assay (Fig. 4A). ii) nuclear translocation of CH is inefficient due to the lack of the NLS resulting in a very low recombination efficiency; this explanation is also unlikely because recombination efficiencies of NCH and CH after transfection were comparable (Fig. 4B) indicating that nuclear translocation is not a limiting factor. This observation is consistent with the previously published finding that addition of a NLS to Cre confers no increase of recombination (Le, Y. et al., Nucleic Acids Res. 27, 4703-9 (1999)). iii) the NLS itself might function as a PTD or at least contributes to the cellular uptake of Cre fusion proteins. In order to investigate the function of NLS during cellular uptake independently of the recombination event we performed an immunohistochemical analysis. Localization of CH and NCH after transduction was monitored by fluorescence microscopy (Fig. 5). NCH was detectable in most of the cells both in the cytoplasm and nucleus, whereas in the case of the CH-treated cells only a low percentage was found to be Cre-positive. Moreover, in the latter case the Cre-specific signals were mainly concentrated in extracellular spot-like structures, whereas NCH staining was more diffuse, dispersing over the whole cell body. Thus, NLS promotes cellular uptake of recombinant Cre.

The comparative analysis of all proteins analyzed in this study demonstrated that the TAT peptide containing HTNC is the most effective protein in transduction and subsequent recombination. One can achieve recombination in more than 90 % of fibroblast reporter cells with only 1 mM HTNC, whereas approx. 5 mM of HNC is necessary to achieve the same level of recombination within the same time (Fig. 3C). Moreover, using HTNC shorter incubation times as compared to HNC are needed to obtain the same recombination efficiency (Fig. 4C). Incubation with 2 mM HTNC for 30 minutes resulted in about 50 % recombination; incubation with 10 mM HNC induced recombination only in approx. 15 % of the cells within the same time. Highly efficient recombination of about 80 % is achieved after 2 h incubation with HTNC, whereas 16 h were needed to obtain the same level of recombination using HNC (Fig. 4C). Thus, TAT peptide enhances transduction of His-NLS-Cre.

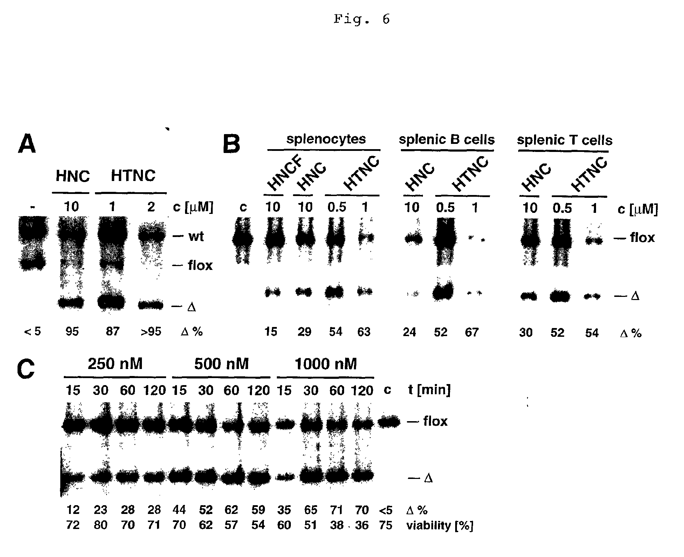

To evaluate further the use of HTNC for genetic engineering we tested transduction into murine ES cells. We used the RDR-IB1N cell line containing a loxP-modified substrate in the ROSA26 locus (F.T. Wunderlich, F.E., K.R., unpublished data). We incubated ES cells in either HNC- or HTNC-containing medium for 20 hours. Southern blot analysis of genomic DNA demonstrated that Cre-mediated recombination was achieved in almost every cell using 2 mM HTNC (Fig. 6A). 87 % deletion was achieved by using only 1 mM HTNC. A 10-fold higher concentration of HNC was necessary to obtain a similar level of deletion. No difference in colony numbers were observed 2 days after transduction between the cell suspensions incubated with 10 mM HNC and 1 mM HTNC, respectively, as compared to control cells incubated in normal medium. A significant decrease of colony numbers was observed in the case of cells treated with more than 2 mM HTNC, indicative of a cytotoxic effect of HTNC at high concentrations. Indeed, growth of cells was almost completely inhibited in the presence of 5 mM HTNC. However, at concentrations which are sufficient to induce deletion in about 90 % of the cells (1-2 mM HTNC) no growth inhibition was observed; Recombination efficiencies of more than 95% after either HTNC- (2 mM) or HNC- (10 mM) transduction were reproducibly confirmed also by employing a different ES-cell line containing a loxP-substrate (data not shown). Additionally, we also wanted to assess the potential of HTNC transduction into resting cells such as primary splenocytes. We therefore incubated splenocytes of mice carrying a loxP-flanked segment in the 5' region of the polb gene (6) either in HNCF-, HNC- or HTNC-containing medium. After 60 minutes of incubation cells were incubated for additional 16 h in normal medium providing time for Cre-mediated recombination. Cells were then sorted into B and T cells. Southern blot analysis of extracted genomic DNAs demonstrated that treatment with 10 mM HNCF results in 15 % deletion in total splenocytes (Fig. 6B, left). As already observed in fibroblasts the same concentration of HNC is more efficient (29 %). However, the most efficient recombination was obtained with 1 mM HTNC (63 %). Similar values were obtained from sorted B and T cells (Fig. 6B, middle and right). 10 mM HNC resulted in 20-30 % recombination, whereas a 10-fold lower concentration of HTNC induced Cre-mediated recombination in 52-67 % of the B and T cells. Even treatment with only 500 nM of HTNC resulted in more than 50 % recombination in either total splenocytes, or sorted B or T cells (Fig. 6B). To assess the time dependency of HTNC-transduction and viability of the treated cells we incubated splenocytes for 15, 30, 60 and 120 minutes in 250 nM, 500 nM as well as 1 mM HTNC (Fig. 6C). Southern analysis revealed that substantial recombination occured already after 15 minutes incubation (12-44 % recombination, depending on the concentration). Recombination efficiency increased with longer incubation times and reached saturation between 30 to 60 minutes. No further increase was observed after 120 minutes. The highest recombination efficiency was obtained after 60 minutes of incubation with 1 mM HTNC (71 %); cell viability of this population was 38 %. Higher viabilities were obtained with lower concentrations: after 60 minutes in 500 nM HTNC 57 % of the splenocytes were still viable and 70 % viability was determined after incubation with 250 nM HTNC; this value is comparable to cells cultured in normal medium displaying a viability of 75 % (Fig. 6C).

### Conclusions:

The aim of this study was to evaluate the potency of two prominent PTDs, namely the hydrophobic FGF as well as the basic TAT peptide, to promote cellular uptake of recombinant Cre. Transduction of His-NLS-Cre and subsequent recombination of a loxP-modified substrate in reporter cells turned out to be independent of the FGF peptide. This observation was unexpected; recently it has been reported that a cell permeable Cre, namely His-NLS-Cre-MTS which is almost identical to the HNCF protein used in our study, can enter mammalian cells and perform recombination (Jo, D, et al., Nat. Biotechnol. 19, 929-933 (2001)). However, a direct comparison of His-NLS-Cre-MTS with a corresponding fusion protein lacking the supposed transduction domain as a proof of the transduction potential of the FGF peptide, has not yet been reported.

If not the FGF peptide, what else then drives the translocation of the His-NLS-Cre across the cellular membrane? The only recombinant protein analyzed in this study displaying no or weak transduction potential was Cre-His, i.e. the only construct without NLS. From this observation we conclude that the basic NLS (see legend of Fig 2A) itself can function as a PTD at least in the context of Cre recombinase. This conclusion is consistent with the observation that basic peptides in general are able to enhance cellular uptake of heterologous proteins or peptides (Wender, P.A. et al., Proc. Natl. Acad. Sci. USA 97, 13003-13008 (2000); Matsushita, M. et al., J. Neurosi. 21, 6000-6007 (2001); Han, K. et al., Mol. Cells 12, 267-271 (2001)). We further extended this observation by generating a fusion of NLS-Cre with the longer basic TAT-derived peptide, known to act very efficiently as a PTD in some experimental settings (Schwarze, S.R. et al., Tr. Cell Biol. 10, 290-295 (2000); Nagahara, H. et al., Nat. Med. 4, 1449-1452 (1998); Schwarze, S.R. et al., Science 285, 1569-1572 (1998)). Indeed, our studies demonstrated that the TAT-modified variant His-TAT-NLS-Cre transduced much more efficiently than His-NLS-Cre. More than 95 % of fibroblast cells as well as murine ES cells undergo Cre-mediated recombination after treatment with 2 mM HTNC. Also primary splenocytes are well accessible to HTNC transduction: recombination efficiencies of about 70 % can be achieved after incubation in 1mM HTNC. Thus, application of HTNC turns out to be a powerful tool to rapidly and efficiently perform Cre-mediated recombination in cultured mammalian cells.

In order to examine the potential of HTNC transduction also in vivo we performed a series of experiments in mice employing HTNC, HNC as well as HNCF. To rule out the possibility that an indirect read-out system (e.g. Cre-mediated activation of a reporter gene) could lead to an overestimation of the actual recombination efficiency, we decided to directly assess the recombination product at the DNA level by Southern analysis. Although using three different mouse lines each containing different loxP-modified loci we did not detect any significant Cre-mediated recombination by Southern blot after intraperitoneal injection of HTNC, HNC and HNCF, respectively. We only observed a weak signal representing approx. 10 % deletion in a single mouse in the peritoneum close to the site of injection (data not shown). We thus failed to induce substantial Cre-mediated recombination in mice by Cre transduction - not even using the very efficient HTNC protein. Recently it has been reported that intraperitoneal administration of His-NLS-Cre-MTS, which is almost identical to HNCF, results in efficient recombination also in mice (Jo, D. et al., Nat. Biotechnol. 19, 929-933 (2001)). However, recombination was determined only indirectly by activation of a LacZ reporter gene. We infer that there might be some, but only poor recombination activity in vivo, especially in local sections or organs which are well-supplied with blood. This putatively minor activity could be strong enough to activate reporter genes in some experimental settings but is insufficient to result in recombination of the loxP targets in the majority of the cells.

In conclusion, HTNC turned out to be widely applicable in cultured mammalian cells resulting in high recombination efficiencies. Every cell line examined in this study, either primary cells ex vivo or stable cell lines, can be modified genetically by Cre transduction with at least a 70% (and up to 95%) efficiency, depending on the cell type. We expect that HTNC transduction will be applicable for a much larger variety of cell lines, thereby overcoming limitations of conventional techniques such as transfection and adenoviral infection. Since HTNC can be readily produced in and purified from *E. coli* in large quantities, we expect HTNC to serve as a powerful tool for genetic manipulation in mammalian cells.

The reporter cell line CV1-5B was provided by G. Schütz.

### Detailed description of the figures 2 to 6:

Fig. 2: Design of expression cassettes and purification of Cre fusion proteins. (A) Six expression constructs were generated. All constructs encode Cre recombinase and a His-tag as represented by white boxes. Black boxes represent PTDs (aa sequence): FGF (AAVLLPVLLAAP) and TAT (GRKKRRQRRR). The grey box represents a NLS (KKKRKV) derived from SV40. Abbreviations of the constructs are given on the left. (B) SDS-PAGE analysis of the purification of HTNC employing Ni(II)-affinity chromatography. Abbreviations used: CL, cleared lysate; FT, flow through; W, wash fraction, E, eluted fraction; M, marker. (C) Comparison of purified fusion proteins on a coomassie stained gel. (D) Immunoblot analysis of purified Cre fusion proteins employing an anti-Cre antibody.
Fig. 3: Transduction of Cre-fusion proteins into a fibroblast Cre reporter cell line. (A) Schematic representation of the reporter gene construct of the fibroblast reporter cell line CV1-5B (Kellendonk, C. et al., Nucleic Acids Res. 24, 1404-1411 (1996)). Cre deletes a loxP-flanked segment thereby activating a LacZ gene. (B) CV1-5B cells were incubated for 16 h in medium containing 2.5 µM (approx. 100 ng/µl) purified Cre fusion proteins as indicated; as a control cells were incubated with normal medium. After incubation cells were washed, incubated with normal medium, fixed and stained for β-gal activity. (C) Quantification of β-gal activities of reporter cells incubated for 16 h with various concentrations of Cre fusion proteins as indicated. Percentages of blue cells are given. (D,E) Southern blot analysis of transduction and subsequent recombination. (D) Time kinetics of HNCF and HNC transduction. Reporter cells were incubated with either 12 µM HNCF or HNC for indicated periods of time. (E) Concentration dependency of HNCF and HNC transduction. Reporter cells were incubated for 16 hours with increasing concentrations of either HNCF or HNC as indicated. Calculated deletion efficiencies in % are given at bottom (Δ%). flox, loxP-flanked gene; Δ, deleted gene.
Fig. 4: (A) Comparison of Cre recombinase activities of HNCF, NCH and CH using a cell-free activity assay. 80 ng of either HNCF, NCH or CH were used to recircularize a linearized substrate vector as described (Jo., D. et al., Nat. Biotechnol. 19, 929-933 (2001); Cantor, E.J., Chong, S., Protein Expr. Purif. 22, 135-140 (2001)). The specific activity of a commercially available control was set to 100 % (C+). Further controls were: 80 ng heat-inactivated Cre protein (C-) and no protein (-). Vertical lines represent ranges of values. (B) Quantitative analysis of recombinase activities of NCH and CH after transfection. CV1-5B cells were transfected with corresponding pTriEx expression vectors or a mock control and stained for β-gal activity. Given are mean values of 3 transfections. n.d., not detectable. (C) Comparison of the time dependency of HNC- and HTNC-transduction. CV1-5B cells were incubated with either 10 µM HNC or 2 µM HTNC between 30 minutes and 16 hours as indicated. After incubation cells were washed, incubated with normal medium, stained for β-gal activity and counted. Percentages of blue cells are given.
Fig. 5: Detection of Cre in cells after transduction employing an anti-Cre antibody. CV1-5B cells were incubated for 4 hours with 5 µM of either CH or NCH or medium only as a control. After transduction cells were examined for immunofluorescence using anti-Cre antiserum or by phase contrast microscopy. Cells were counter-stained using 4,6-di-amino-2-phenylindol (DAPI). See *Materials and Methods* for details.
Fig. 6: HTNC transduction into (A) murine embryonic stem cells and (B,C) splenocytes ex vivo analyzed by Southern blot. (A) RDR-IB1N ES cells were incubated for 20 hours in medium containing indicated concentrations of either HNC or HTNC. After 3 additional days of incubation in normal ES-medium genomic DNAs were examined by Southern-blot analysis. (B) Splenocytes of a polβ-flox/flox mouse (Gu., H. et al., Science 265, 103-106 (1994)) were incubated 60 minutes in medium containing indicated concentrations of either HNCF, HNC or HTNC. After transduction cells were incubated for 16 h in normal medium. Shown is Southern blot analysis of sorted B cells (CD19⁺/PI⁻), T cells (Thy1.2⁺/ PI⁻) as well as total splenocytes. (C) Splenocytes of a polβ-flox mouse were incubated with medium containing 250 nM, 500 nM and 1000 nM of HTNC for indicated periods of time. Calculated deletion efficiencies (Δ%) and cell viabilities in % are given at the bottom. wt, wild type allele; flox, loxP-flanked allele; Δ, deleted allele.

## Claims

1. A fusion protein comprising
(a) a site-specific DNA recombinase domain,
(b) a domain containing a modified nuclear localization signal (NLS) of type one or two which differs from a natural, Pro-containing in that it lacks the Pro residue(s).

2. The fusion protein of claim 1, wherein the site-specific DNA recombinase domain is selected from a recombinase protein derived from Cre, Flp, φC31 recombinase, and R recombinase and preferably is Cre having amino acids 15 to 357 of SEQ ID NO: 2, Flpe having amino acids 15 to 437 of SEQ ID NO: 4, or a fragment or derivative thereof having equivalent recombinase activity.

3. The fusion protein of claim 1 or 2, wherein the NLS is a type one NLS having 5 to 10 amino acid residues and containing at least 5 basic amino acid residues and no Pro residue, preferably the NLS has the sequence RKKRKV (SEQ ID NO:6)

4. The fusion protein according to any one of claims 1 to 3, wherein
(i) domains (a) and (b) are directly or through a linker peptide linked to each other; and/or
(ii) the domain (b) is linked to the N-terminal of the domain (a).

5. The fusion protein of claim 1, which has the amino acid sequence shown in SEQ ID NO:37 or 39

6. The fusion protein according to any one of claims 1 to 5, which further comprises
(c) a protein transduction domain (PTD), preferably said PTD is derived from Antennapedia, from the VP22 protein of HSV, from the TAT protein of HIV or from the Karposi fibroblast growth factor (FGF), or is a derivative thereof, and/or
(d) non-NLS and non-PTD functional sequences which are preferably selected from secretion conferring signals, signals conferring protein stabilization, signals facilitating purification, etc.

7. The fusion protein of claim 6, wherein the PTD is
(i) TAT having the amino acid sequence YGRKKRRQRRR (SEQ ID NO: 7) or a mutant thereof including peptides having the amino sequences GRKKRRQRRR (SEQ ID NO:8) AGRKKRRQRRR (SEQ ID NO:9) YARKARRQARR (SEQ ID NO:10) YARAAARQARA (SEQ ID NO:11) YARAARRAARR (SEQ ID NO:12) YARAARRAARA (SEQ ID NO:13) YARRRRRRRRR (SEQ ID NO:14) YAAARRRRRRR (SEQ ID NO:15); preferably the TAT protein consists of one of said sequences;
(ii) a VP22 protein comprising the amino acid 16-157 of SEQ ID NO:17, preferably the VP22 protein consists of said sequence; or
(iii) a FGF having the amino acid sequence AAVLLPVLLAAP (SEQ ID NO:18).

8. The fusion protein of claim 6 or 7, wherein the domain (c)
(i) is fused to domain (a) and/or domain (b) through a direct chemical bond or through a linker molecule; and/or
(ii) is fused to the N-terminal of domain (a) or to the N-terminal of domain (b) which in turn being linked to the N-terminal of domain (a).

9. The fusion protein of claim 6 which has the sequence shown in SEQ ID NO:41.

10. The fusion protein of claim 4 or 8, wherein the linker molecule is a short peptide having 1 to 20, preferably 1 to 10 amino acid residues.

11. Use of a fusion protein as defined in claims 1 to 10 for preparing an agent for inducing target gene alterations in living organisms or in cell cultures, wherein said living organisms or cells of said cell cultures carry at least one or more recognition sites for said site-specific DNA recombinase integrated in its genome.

12. Use of a fusion protein comprising
(a) a site specific DNA recombinase domain
(b') a domain containing a nuclear localisation signal (NLS) of type one, two or three
for preparing an agent for inducing target gene alterations in living organisms or cell cultures, wherein said living organisms or in cells of said cell cultures carry at least one or more recognition sites for said site-specific DNA recombinase integrated in its genome.

13. The use of claim 1 wherein
(i) the domain (a) is as defined in claim 2; and/or
(ii) the domain (b') comprises the sequence of SEQ ID NO:5; and/or
(iii) domains (a) and (b') are directly or through a linker peptide linked to each other; and/or
(iv) the domain (b') is linked to the N-terminal of the domain (a).

14. The use of claim 11, 12 or 13, wherein the recognition sites for said site specific recombinase is present within an endogenous gene or a transgene.

15. The use of any one of claims 11 to 14, wherein the living organism is a vertebrate, preferably a rodent or a fish, or when the cells of the cell culture are derived from vertebrates, preferably rodent or fish.

16. A method for inducing gene alterations in living organisms or in cell cultures, which method comprises administering to said living organisms or to cells of said cell cultures a fusion protein as defined in claims 1 to 10, wherein said living organisms or cells of said cell cultures carry at least one or more recognition sites for said site-specific DNA recombinase integrated in its genome.

17. A method for inducing gene alterations in living organisms or cell cultures, which method comprises administering to said living organisms or to cells of said cell cultures a fusion protein as defined in claim 12 or 13, wherein said living organisms carry at least one or more recognition sites for said site-specific DNA recombinase integrated in its genome.

18. A DNA sequence coding for the fusion protein as defined in claims 1 to 10, said DNA sequence preferably comprising the sequence shown in SEQ ID NOs:1, 3, 36, 38 and/or 40.

19. A vector comprising the DNA sequence of claim 18.

20. A host cell transformed with the vector of claim 19 and/or comprising the DNA of claim 18.

21. A transgene organism containing the DNA of claim 18 in its genome.

22. A method for producing the fusion protein of claim 1 which comprises culturing the transformed host cell of claim 20 and isolating the fusion protein.

23. An injectable composition comprising the fusion protein as defined in claims 1 to 10.

24. A method for inducing gene alterations in a living organisms or cell cultures which comprises
(i) transfection or viral infection of living organisms or cell cultures with a DNA as defined in claim 18 or a vector as defined in claim 19, or
(ii) preparing transgene living organisms or cell cultures containing a DNA sequence as defined in claim 18 integrated in its genome, wherein said living organisms or cells of said cell culture carry at least one or more recognition sites for said site-specific DNA recombinase integrated in its genome.

25. Use of a modified localization signal (NLS) as defined in claim 1 or 3 for enhancing cellular uptake of functional biopolymers in living organisms or cell cultures.
